# EUROPEAN PATENT APPLICATION

(11) **EP 4 692 041 A1**
(43) Date of publication of application: **11.02.2026**
(21) Application number: 24780494.1
(22) Date of filing: 27.03.2024
(51) Int. Cl.: C07C 67/08, C07B 61/00, C07C 29/34, C07C 31/125, C07C 69/24, C07C 69/84

(54) **METHOD FOR PRODUCING BIO-DERIVED ESTER COMPOUND, BIO-DERIVED ESTER COMPOUND PRODUCED THEREBY, COSMETIC OR DETERGENT CONTAINING BIO-DERIVED ESTER COMPOUND, AND METHOD FOR REDUCING ODOR OF ESTER COMPOUND**

(30) Priority: 30.03.2023 JP 2023055220
(71) Applicant: ADEKA CORPORATION, Arakawa-ku Tokyo 116-8554 (JP)
(72) Inventor: INOUE, Takahiro, Tokyo 116-8554 (JP); HORI, Aruto, Tokyo 116-8554 (JP); TAKATA, Masahiro, Tokyo 116-8554 (JP)
(74) Representative: Müller-Boré & Partner Patentanwälte PartG mbB
(86) International application number: PCT/JP2024/012325
(87) International publication number: WO 2024/204392

(57) **Abstract**

The present invention provides a method of producing a bio-derived ester compound with reduced odor, a bio-derived ester compound produced by the production method, a cosmetic or a detergent including the ester compound, and a method of reducing an odor of an ester compound.

## Description

### Technical Field

The present invention relates to a method of producing an ester compound, an ester compound, and a method of reducing an odor of an ester compound. Specifically, the present invention relates to a method of producing a bio-derived ester compound, which can provide a bio-derived ester compound with reduced odor by using a bio-derived raw material.

### Background Art

In a cosmetic, a detergent, and the like, it has been known that various ester compounds are used in order to provide a composition having desired characteristics (e.g., Patent Documents 1 to 3). As a method of producing such ester compound, there has been known, for example, a method involving causing a carboxylic acid and a monohydric alcohol to react with each other, as described in Patent Documents 4 and 5.

### Citation List

### Patent Document

[Patent Document 1] JP 2010-539189 A
[Patent Document 2] JP 2022-163970 A
[Patent Document 3] JP 2014-122195 A
[Patent Document 4] JP 48-54016 A
[Patent Document 5] JP 49-11815 A

### Summary of Invention

### Technical Problem

However, even in the above-mentioned production method, there has been a problem in that, for example, an ester compound having an odor may be obtained. In addition, in recent years, in the field of chemicals in general including cosmetic ingredients, there has been a demand that part or all of raw materials to be used in the production of chemicals be raw materials (bio-derived raw materials) derived from living organisms, such as animals or plants, from the viewpoints of environmental friendliness and the like. Accordingly, an object of the present invention is to provide a method of producing a bio-derived ester compound, which can provide a bio-derived ester compound with reduced odor while using a bio-derived raw material.

### Solution to Problem

In view of the foregoing, the inventors of the present invention have made intensive investigations, and have completed the present invention. That is, according to one embodiment of the present invention, there is provided a method of producing a bio-derived ester compound, comprising: a step of providing a bio-derived branched primary alcohol having a branched alkyl group having 6 to 12 carbon atoms, the step comprising dimerizing one kind or two or more kinds selected from the group consisting of: a bio-derived linear primary alcohol having a linear alkyl group having 3 to 6 carbon atoms; a bio-derived linear primary alcohol having a linear alkenyl group having 3 to 6 carbon atoms; a bio-derived linear aldehyde having a linear alkyl group having 3 to 6 carbon atoms; and a bio-derived linear aldehyde having a linear alkenyl group having 3 to 6 carbon atoms; and a step of providing a bio-derived ester compound by using the resultant bio-derived branched primary alcohol having 6 to 12 carbon atoms and a carboxylic acid-based compound having a hydrocarbon group having 1 to 22 carbon atoms or an anhydride thereof.

In addition, according to another embodiment of the present invention, there is provided a bio-derived ester compound, which is produced by the above-mentioned production method.

Further, according to another embodiment of the present invention, there is provided a cosmetic or a detergent, comprising the above-mentioned ester compound.

Further, according to another embodiment of the present invention, there is provided a method of reducing an odor of an ester compound, comprising causing a bio-derived branched primary alcohol having a branched alkyl group having 6 to 12 carbon atoms to react as a raw material.

### Advantageous Effects of Invention

The present invention can provide the method of producing a bio-derived ester compound, which can provide a bio-derived ester compound with reduced odor by using a bio-derived raw material.

### Description of Embodiments

A bio-derived linear primary alcohol to be used in the present invention refers to one kind or two or more kinds selected from the group consisting of: a bio-derived linear primary alcohol having a linear alkyl group having 3 to 6 carbon atoms, which is obtained from a plant resource or the like (hereinafter sometimes simply referred to as "bio-derived"), and which has a structure in which one hydrogen atom bonded to a carbon atom at one terminal of a linear alkane having 3 to 6 carbon atoms is substituted with one hydroxy group; and a bio-derived linear primary alcohol having a linear alkenyl group having 3 to 6 carbon atoms, which is obtained from a plant resource or the like, and which has a structure in which one hydrogen atom bonded to a carbon atom at one terminal of a linear alkene having 3 to 6 carbon atoms is substituted with one hydroxy group. Specific examples of the bio-derived linear primary alcohol having a linear alkyl group having 3 to 6 carbon atoms out of those bio-derived linear primary alcohols include bio-derived n-propanol (1-propanol), bio-derived n-butanol (1-butanol), bio-derived n-pentanol (1-pentanol), and bio-derived n-hexanol (1-hexanol). In addition, specific examples of the bio-derived linear primary alcohol having a linear alkenyl group having 3 to 6 carbon atoms include bio-derived n-propenyl alcohol (allyl alcohol), bio-derived n-butenyl alcohol (crotyl alcohol), bio-derived n-pentenyl alcohol, and bio-derived n-hexenyl alcohol. A bio-derived linear primary alcohol obtained by a known method may be used as such bio-derived linear primary alcohol. For example, there may be used: a bio-derived linear primary alcohol obtained by subjecting biomass derived from corn, sugar cane, sugar beet, banana, wheat, barley, rye, potato, sweet potato, cassava, taro, broad beans, lentils, peas, or the like to fermentation and/or metabolism with a microorganism; and a bio-derived linear primary alcohol obtained by synthesis using, as a raw material, any of various bio-derived compounds each obtained from a plant resource or the like, such as palm oil, palm kernel oil, soybean oil, rapeseed oil, castor oil, olive oil, cottonseed oil, coconut oil, corn oil, safflower oil, sesame oil, sunflower oil, camellia oil, or linseed oil.

There is no particular limitation on a method of providing a bio-derived linear primary alcohol by fermenting biomass derived from corn, sugar cane, sugar beet, banana, wheat, barley, rye, potato, sweet potato, cassava, taro, broad beans, lentils, peas, or the like with a microorganism, and a known method may be used. For example, there may be used a method involving fermenting and/or metabolizing a saccharide such as cellulose obtained from biomass derived from corn, sugar cane, sugar beet, banana, wheat, barley, rye, potato, sweet potato, cassava, taro, broad beans, lentils, peas, or the like with a microorganism, such as a microbe, an enzyme, or yeast, having a fermenting and/or metabolizing ability under conditions of an appropriate temperature, humidity, atmosphere, and the like.

In addition, there is no particular limitation on a method of providing a bio-derived linear primary alcohol by synthesis using, as a raw material, any of various bio-derived compounds each obtained from a plant resource or the like, such as palm oil, palm kernel oil, soybean oil, rapeseed oil, olive oil, cottonseed oil, or coconut oil, and a known method may be used. For example, there may be used: a method involving methylesterifying a fatty acid obtained by hydrolyzing a fatty acid glyceride in a vegetable oil, such as palm oil, palm kernel oil, soybean oil, rapeseed oil, castor oil, olive oil, cottonseed oil, coconut oil, corn oil, safflower oil, sesame oil, sunflower oil, camellia oil, or linseed oil, followed by hydrogenation; or a method involving hydrogenating a bio-derived linear aldehyde having a linear alkyl group having 3 to 6 carbon atoms or a bio-derived linear aldehyde having a linear alkenyl group having 3 to 6 carbon atoms by a known method. In this case, a bio-derived linear primary alcohol having a hydrocarbon group having the number of carbon atoms different from that of the bio-derived compound to be used as the raw material may be produced. For example, in the present invention, there may be used a bio-derived linear primary alcohol increased in number of carbon atoms as compared to the hydrocarbon group in the bio-derived alcohol used as the raw material, the bio-derived linear primary alcohol being obtained by oxidizing or dehydrogenating a bio-derived alcohol, such as ethanol or propanol, serving as a raw material by a known method to provide an aldehyde compound, and then condensing or hydrogenating the resultant aldehyde compound.

In the bio-derived linear primary alcohol to be used in the present invention, it is not required that all of carbon atoms for forming the bio-derived linear primary alcohol be bio-derived. However, from the viewpoints of environmental friendliness, ease of production, and the like, it is preferred that 50% or more of the carbon atoms for forming the bio-derived linear primary alcohol be bio-derived carbon atoms, and it is more preferred that all of the carbon atoms for forming the bio-derived linear primary alcohol be bio-derived carbon atoms.

One kind or two or more kinds of the bio-derived linear primary alcohols described above may each be used as the bio-derived linear primary alcohol to be used in the present invention. However, from the viewpoint of ease of production of a single bio-derived ester compound, it is preferred that only one kind of bio-derived linear primary alcohol be used. In addition, in the present invention, from the viewpoint of providing a bio-derived ester compound with further reduced odor, bio-derived n-butanol (1-butanol) is preferably used as the bio-derived linear primary alcohol, and only bio-derived n-butanol is more preferably used.

A bio-derived linear aldehyde to be used in the present invention refers to one kind or two or more kinds selected from the group consisting of: a bio-derived linear aldehyde having a linear alkyl group having 3 to 6 carbon atoms, which is obtained from a plant resource or the like, and which has a structure in which a methyl group at one terminal of a linear alkane having 3 to 6 carbon atoms is substituted with one aldehyde group; and a bio-derived linear aldehyde having a linear alkenyl group having 3 to 6 carbon atoms, which is obtained from a plant resource or the like, and which has a structure in which a methyl group at one terminal of a linear alkene having 3 to 6 carbon atoms is substituted with one aldehyde group. Specific examples of the bio-derived linear aldehyde having a linear alkyl group having 3 to 6 carbon atoms out of those bio-derived linear aldehydes include bio-derived n-propionaldehyde (propanal), bio-derived n-butyraldehyde (butanal), bio-derived n-valeraldehyde (pentanal), and bio-derived n-hexylaldehyde (hexanal). In addition, specific examples of the bio-derived linear aldehyde having a linear alkenyl group having 3 to 6 carbon atoms include bio-derived n-propene aldehyde (acrolein), bio-derived n-propylene aldehyde (butenal, crotonaldehyde), bio-derived n-butylene aldehyde (pentenal), and bio-derived n-pentene aldehyde (hexanal). A bio-derived linear aldehyde obtained by a known method may be used as such bio-derived linear aldehyde. For example, there may be used: a bio-derived linear aldehyde obtained by subjecting biomass derived from corn, sugar cane, sugar beet, banana, wheat, barley, rye, potato, sweet potato, cassava, taro, broad beans, lentils, peas, or the like to fermentation and/or metabolism with a microorganism; and a bio-derived linear aldehyde obtained by synthesis using, as a raw material, any of various bio-derived compounds obtained from a plant resource or the like, such as palm oil, palm kernel oil, soybean oil, rapeseed oil, castor oil, olive oil, cottonseed oil, coconut oil, corn oil, safflower oil, sesame oil, sunflower oil, camellia oil, or linseed oil.

There is no particular limitation on a method of providing a bio-derived linear aldehyde by fermenting biomass derived from corn, sugar cane, sugar beet, banana, wheat, barley, rye, potato, sweet potato, cassava, taro, broad beans, lentils, peas, or the like with a microorganism, and a known method may be used. For example, there may be used a method involving fermenting and/or metabolizing a saccharide such as cellulose obtained from biomass derived from corn, sugar cane, sugar beet, banana, wheat, barley, rye, potato, sweet potato, cassava, taro, broad beans, lentils, peas, or the like with a microorganism, such as a microbe, an enzyme, or yeast, having a fermenting and/or metabolizing ability under conditions of an appropriate temperature, humidity, atmosphere, and the like.

In addition, there is no particular limitation on a method of providing a bio-derived linear aldehyde by synthesis using, as a raw material, any of various bio-derived compounds obtained from a plant resource or the like, such as palm oil, palm kernel oil, soybean oil, rapeseed oil, olive oil, cottonseed oil, or coconut oil, and a known method may be used. In this case, a bio-derived linear aldehyde having a hydrocarbon group having the number of carbon atoms different from that of the bio-derived compound to be used as the raw material may be produced so as to be used in the present invention. Examples of such method include: a method involving, by a known method, oxidizing or dehydrogenating a bio-derived linear primary alcohol having a linear alkyl group having 3 to 6 carbon atoms or a bio-derived linear primary alcohol having a linear alkenyl group having 3 to 6 carbon atoms obtained by a known method; a method involving performing hydroformylation by an oxo method using a bio-derived alkene having 2 to 5 carbon atoms, such as bio-derived ethylene, bio-derived propylene, bio-derived butylene, or bio-derived pentene obtained by a known method, carbon monoxide, and hydrogen; and a method involving condensing an aldehyde having 2 to 4 carbon atoms, which is obtained by oxidizing or dehydrogenating a bio-derived alcohol, such as ethanol or propanol, by a known method, to produce an aldehyde having an increased number of carbon atoms.

In the bio-derived linear aldehyde to be used in the present invention, it is not required that all of carbon atoms for forming the bio-derived linear aldehyde be bio-derived. However, from the viewpoints of environmental friendliness, ease of production, and the like, it is preferred that 50% or more of the carbon atoms for forming the bio-derived linear aldehyde be bio-derived carbon atoms, and it is more preferred that all of the carbon atoms for forming the bio-derived linear aldehyde be bio-derived carbon atoms.

One kind or two or more kinds of the bio-derived linear aldehydes described above may each be used as the bio-derived linear aldehyde to be used in the present invention. However, from the viewpoint of ease of production of a single bio-derived ester compound, it is preferred that one kind of bio-derived linear aldehyde be used. In addition, in the present invention, from the viewpoint of easily providing a bio-derived ester compound with further reduced odor, bio-derived n-butyraldehyde (butanal) is preferably used as the bio-derived linear aldehyde.

There is no particular limitation on a method of dimerizing one kind or two or more kinds selected from the group consisting of: the bio-derived linear primary alcohol having a linear alkyl group having 3 to 6 carbon atoms; the bio-derived linear primary alcohol having a linear alkenyl group having 3 to 6 carbon atoms; the bio-derived linear aldehyde having a linear alkyl group having 3 to 6 carbon atoms; and the bio-derived linear aldehyde having a linear alkenyl group having 3 to 6 carbon atoms, and a known method may be used as each method. For example, a method of dimerizing an alcohol described in Catal. Sci. Technol., 2015, vol. 5, p. 3876-3902, or a method of dimerizing an aldehyde described in JP 09-124536 A may be used. Specifically, for example, there may be used a method of dimerizing the bio-derived linear primary alcohols each having a linear alkyl group having 3 to 6 carbon atoms, the bio-derived linear primary alcohols each having a linear alkenyl group having 3 to 6 carbon atoms, the bio-derived linear aldehydes each having a linear alkyl group having 3 to 6 carbon atoms, or the bio-derived linear aldehydes each having a linear alkenyl group having 3 to 6 carbon atoms through a reaction at room temperature or under heated condition, under reduced pressure , under ambient pressure, or under increased pressure, in the presence of a catalyst, a solvent, or the like as required. In addition, in the present invention, there may be used a method of dimerizing each of the bio-derived linear primary alcohols and the bio-derived linear aldehydes through a reaction under the above-mentioned conditions in a system in which two or more kinds selected from the group consisting of: the bio-derived linear primary alcohol having a linear alkyl group having 3 to 6 carbon atoms; the bio-derived linear primary alcohol having a linear alkenyl group having 3 to 6 carbon atoms; the bio-derived linear aldehyde having a linear alkyl group having 3 to 6 carbon atoms; and the bio-derived linear aldehyde having a linear alkenyl group having 3 to 6 carbon atoms are mixed. Further, there may be used a method of dimerizing each of the same bio-derived linear primary alcohols and the same bio-derived linear aldehydes through a reaction. For example, a bio-derived branched primary alcohol may be provided through a step of dimerizing bio-derived propanols, bio-derived n-butanols, bio-derived n-pentanols, or bio-derived n-hexanols, and a bio-derived branched primary alcohol may be provided through a step of dimerizing bio-derived n-propanals, bio-derived n-butanals, n-pentanals, or n-hexanals.

There is no particular limitation on the catalyst that may be used in the dimerization of the bio-derived linear primary alcohol or the dimerization of the bio-derived linear aldehyde, and a known catalyst may be used in accordance with purposes. Examples thereof include: a metal catalyst, such as powder, an oxide, a complex, a salt, or an alkoxide of a metal, such as copper, silver, zinc, nickel, palladium, platinum, cobalt, rhodium, iridium, iron, calcium, potassium, magnesium, ruthenium, manganese, chromium, or molybdenum; a nitroxyl radical-based catalyst such as 2,2,6,6-tetramethylpiperidine-1-oxyl (TEMPO); and a basic compound, such as an oxide, a hydroxide, a carbonate, a carboxylate, a phosphate, an amine salt, or an alkoxide of an alkali metal, such as lithium, sodium, or potassium, or of an alkaline earth metal, such as magnesium or calcium, and one kind or two or more kinds thereof may be used. Of those, when a metal catalyst is used as the catalyst, a compound serving as a ligand may be used in combination therewith. Examples of such ligand include: an olefin ligand, such as ethylene, norbornene, norbornadiene, 1,7-octadiene, 1,5-cyclooctadiene, or pentamethylcyclopentadienyl; a phosphine ligand, such as ethylenebis(diphenylphosphine), cyclohexyldiphenylphosphine, dicyclohexylphenylphosphine, trit-butylphosphine, or triphenylphosphine; and a nitrogencontaining ligand, such as triethylamine, benzylamine, bipyridyl, bisiminopyridine, or imidazole, and one kind or two or more kinds thereof may be used. In addition, as the basic compound that may be used as the catalyst, the above-mentioned basic compound supported on zeolite, silica, alumina zirconia, magnesia, activated carbon, graphite, a carbon nanotube, or the like may be used.

In the present invention, of those, from the viewpoint of easily providing a bio-derived ester compound with reduced odor, as the catalyst, one kind or two or more kinds selected from the group consisting of: a metal catalyst; and a basic compound are preferably used, one kind or two or more kinds selected from the group consisting of: powder or an oxide of a metal selected from copper, nickel, or palladium; and an oxide, a hydroxide, or a phosphate of sodium, potassium, magnesium, or calcium are more preferably used, one kind or two or more kinds selected from the group consisting of: copper powder; a copper oxide; and an oxide or a phosphate of potassium or calcium are still more preferably used, and one kind or two or more kinds selected from the group consisting of: copper powder; tripotassium phosphate; and calcium oxide are particularly preferably used.

In the present invention, there is no particular limitation on the amount of the catalyst in the case where the catalyst is used in each of the dimerization of the bio-derived linear primary alcohol and the dimerization of the bio-derived linear aldehyde, and the amount of the catalyst may be appropriately adjusted in accordance with purposes. From the viewpoint of efficiently performing the reaction, the usage amount of the catalyst is set to preferably from 0.001 part by mass to 50 parts by mass, more preferably from 0.1 part by mass to 45 parts by mass, still more preferably from 1 part by mass to 40 parts by mass, particularly preferably from 10 parts by mass to 35 parts by mass when the amount of each of the bio-derived linear primary alcohol and the bio-derived linear aldehyde to be used is set to 100 parts by mass.

In the present invention, from the viewpoint of efficiently performing each of the dimerization reaction of the bio-derived linear primary alcohol and the dimerization reaction of the bio-derived linear aldehyde, the method of dimerizing the bio-derived linear primary alcohols through a reaction and the method of dimerizing the bio-derived linear aldehydes through a reaction are preferably a method of dimerizing the bio-derived linear primary alcohols through a reaction in the presence of a catalyst and a method of dimerizing the bio-derived linear aldehydes through a reaction in the presence of a catalyst, respectively.

There is no particular limitation on the solvent that may be used in each of the dimerization of the bio-derived linear primary alcohol and the dimerization of the bio-derived linear aldehyde, and a known solvent may be used in accordance with purposes. Examples of the solvent include water, pentane, hexane, heptane, octane, decane, dodecane, benzene, toluene, xylene, ethylbenzene, dodecylbenzene, diethylene glycol dimethyl ether, triethylene glycol dimethyl ether, diphenyl ether, dibenzyl ether, diallyl ether, tetrahydrofuran, dioxane, N-methyl-2-pyrrolidone, ethyl butyrate, butyl butyrate, ethyl acetate, butyl acetate, dimethylformamide, N,N-dimethylacetamide, acetonitrile, propionitrile, and benzonitrile, and one kind or two or more kinds thereof may be used.

In the present invention, there is no particular limitation on the amount of the solvent in the case where the solvent is used in each of the dimerization of the bio-derived linear primary alcohol and the dimerization of the bio-derived linear aldehyde, and the amount of the solvent may be appropriately adjusted in accordance with purposes. From the viewpoint of efficiently performing the reaction, the usage amount of the solvent is set to preferably from 1 part by mass to 500 parts by mass, more preferably from 10 parts by mass to 300 parts by mass, still more preferably from 3 part by mass to 200 parts by mass when the amount of each of the bio-derived linear primary alcohol and the bio-derived linear aldehyde to be used is set to 100 parts by mass.

In the present invention, from the viewpoint of simply performing each of the dimerization reaction of the bio-derived linear primary alcohol and the dimerization reaction of the bio-derived linear aldehyde, the method of dimerizing the bio-derived linear primary alcohols through a reaction and the method of dimerizing the bio-derived linear aldehydes through a reaction are preferably a method of dimerizing the bio-derived linear primary alcohols through a reaction in the absence of a solvent and a method of dimerizing the bio-derived linear aldehydes through a reaction in the absence of a solvent, respectively. Particularly in this case, a method of dimerizing the same bio-derived linear primary alcohols through a reaction or a method of dimerizing the same bio-derived linear aldehydes through a reaction is more preferred.

The temperature in each of the dimerization of the bio-derived linear primary alcohol and the dimerization of the bio-derived linear aldehyde is not particularly limited as long as the bio-derived linear primary alcohol or the bio-derived linear aldehyde can be dimerized at the temperature, and may be appropriately adjusted in accordance with purposes. For example, the temperature may be set to from room temperature to 340°C. From the viewpoint of easily providing a bio-derived ester compound with reduced odor, the temperature is set to preferably from 60°C to 330°C, more preferably from 120°C to 320°C, still more preferably from 180°C to 310°C, particularly preferably from 240°C to 300°C out of those temperatures.

In the present invention, from the viewpoint of efficiently performing each of the dimerization reaction of the bio-derived linear primary alcohol and the dimerization reaction of the bio-derived linear aldehyde, the method of dimerizing the bio-derived linear primary alcohols through a reaction and the method of dimerizing the bio-derived linear aldehydes through a reaction are preferably a method of dimerizing the bio-derived linear primary alcohols through a reaction under heated conditions and a method of dimerizing the bio-derived linear aldehydes through a reaction under heated conditions, respectively. Particularly in this case, a method of dimerizing the same bio-derived linear primary alcohols through a reaction or a method of dimerizing the same bio-derived linear aldehydes through a reaction is more preferred.

The reaction time in each of the dimerization of the bio-derived linear primary alcohol and the dimerization of the bio-derived linear aldehyde is not particularly limited and may be appropriately adjusted in accordance with purposes. For example, the reaction time may be set to from 10 minutes to 72 hours. From the viewpoint of easily providing a bio-derived ester compound with reduced odor, the reaction time is set to preferably from 30 minutes to 48 hours, more preferably from 1 hour to 24 hours out of those reaction times.

In the present invention, when at least one of the dimerization of the bio-derived linear primary alcohol or the dimerization of the bio-derived linear aldehyde is performed under reduced pressure, the pressure is not particularly limited and may be appropriately adjusted in accordance with purposes. For example, the dimerization may be performed under a pressure of from 0.01 Pa to 80,000 Pa. In addition, when at least one of the dimerization of the bio-derived linear primary alcohol and the dimerization of the bio-derived linear aldehyde is performed under increased pressure, the pressure is not particularly limited and may be appropriately adjusted in accordance with purposes. For example, the dimerization may be performed at a pressure of from 0.001 MPa to 10 MPa with respect to the atmospheric pressure.

In the present invention, from the viewpoint of simply performing each of the dimerization reaction of the bio-derived linear primary alcohol and the dimerization reaction of the bio-derived linear aldehyde, the method of dimerizing the bio-derived linear primary alcohols through a reaction and the method of dimerizing the bio-derived linear aldehydes through a reaction are preferably a method of dimerizing the bio-derived linear primary alcohols through a reaction under ambient pressure and a method of dimerizing the bio-derived linear aldehydes through a reaction under ambient pressure, respectively. Particularly in this case, a method of dimerizing the same bio-derived linear primary alcohols through a reaction or a method of dimerizing the same bio-derived linear aldehydes through a reaction is more preferred.

In the present invention, from the viewpoint of simply and efficiently performing each of the dimerization reaction of the bio-derived linear primary alcohol and the dimerization reaction of the bio-derived linear aldehyde, the method of dimerizing the bio-derived linear primary alcohols through a reaction and the method of dimerizing the bio-derived linear aldehydes through a reaction are more preferably a method of dimerizing the bio-derived linear primary alcohols through a reaction in the presence of a catalyst and in the absence of a solvent and a method of dimerizing the bio-derived linear aldehydes through a reaction in the presence of a catalyst and in the absence of a solvent, respectively. Particularly in this case, a method of dimerizing the same bio-derived linear primary alcohols through a reaction or a method of dimerizing the same bio-derived linear aldehydes through a reaction is still more preferred.

In the present invention, from the viewpoint of simply and efficiently performing each of the dimerization reaction of the bio-derived linear primary alcohol and the dimerization reaction of the bio-derived linear aldehyde, the method of dimerizing the bio-derived linear primary alcohols through a reaction and the method of dimerizing the bio-derived linear aldehydes through a reaction are more preferably a method of dimerizing the bio-derived linear primary alcohols through a reaction in the presence of a catalyst and under ambient pressure and a method of dimerizing the bio-derived linear aldehydes through a reaction in the presence of a catalyst and under ambient pressure, respectively. Particularly in this case, a method of dimerizing the same bio-derived linear primary alcohols through a reaction or a method of dimerizing the same bio-derived linear aldehydes through a reaction is still more preferred.

In the present invention, from the viewpoint of efficiently performing each of the dimerization reaction of the bio-derived linear primary alcohol and the dimerization reaction of the bio-derived linear aldehyde, the method of dimerizing the bio-derived linear primary alcohols through a reaction and the method of dimerizing the bio-derived linear aldehydes through a reaction are more preferably a method of dimerizing the bio-derived linear primary alcohols through a reaction in the presence of a catalyst and under heated conditions, and a method of dimerizing the bio-derived linear aldehydes through a reaction in the presence of a catalyst and under heated conditions, respectively. Particularly in this case, a method of dimerizing the same bio-derived linear primary alcohols through a reaction or a method of dimerizing the same bio-derived linear aldehydes through a reaction is still more preferred.

In addition, from the viewpoint of simply and more efficiently performing each of the dimerization reaction of the bio-derived linear primary alcohol and the dimerization reaction of the bio-derived linear aldehyde, the method of dimerizing the bio-derived linear primary alcohols through a reaction and the method of dimerizing the bio-derived linear aldehydes through a reaction are still more preferably a method of dimerizing the bio-derived linear primary alcohols through a reaction in the presence of a catalyst, under heated conditions, and in the absence of a solvent, and a method of dimerizing the bio-derived linear aldehydes through a reaction in the presence of a catalyst, under heated conditions, and in the absence of a solvent, respectively. In this case, a method of dimerizing the same bio-derived linear primary alcohols through a reaction or a method of dimerizing the same bio-derived linear aldehydes through a reaction is particularly preferred.

In addition, from the viewpoint of simply and more efficiently performing each of the dimerization reaction of the bio-derived linear primary alcohol and the dimerization reaction of the bio-derived linear aldehyde, the method of dimerizing the bio-derived linear primary alcohols through a reaction and the method of dimerizing the bio-derived linear aldehydes through a reaction are still more preferably a method of dimerizing the bio-derived linear primary alcohols through a reaction in the presence of a catalyst, under heated conditions, and under ambient pressure, and a method of dimerizing the bio-derived linear aldehydes through a reaction in the presence of a catalyst, under heated conditions, and under ambient pressure, respectively. In this case, a method of dimerizing the same bio-derived linear primary alcohols through a reaction or a method of dimerizing the same bio-derived linear aldehydes through a reaction is particularly preferred.

Of those, in the present invention, from the viewpoint of simply and more efficiently performing each of the dimerization reaction of the bio-derived linear primary alcohol and the dimerization reaction of the bio-derived linear aldehyde, the method of dimerizing the bio-derived linear primary alcohols through a reaction and the method of dimerizing the bio-derived linear aldehydes through a reaction are particularly preferably a method of dimerizing the bio-derived linear primary alcohols through a reaction in the presence of a catalyst, under heated conditions, in the absence of a solvent, and under ambient pressure, and a method of dimerizing the bio-derived linear aldehydes through a reaction in the presence of a catalyst, under heated conditions, in the absence of a solvent, and under ambient pressure, respectively. In this case, a method of dimerizing the same bio-derived linear primary alcohols through a reaction or a method of dimerizing the same bio-derived linear aldehydes through a reaction is most preferred.

When a bio-derived linear primary alcohol having a linear alkyl group having 3 to 6 carbon atoms is used as the bio-derived linear primary alcohol serving as a raw material, it is only necessary that the step of providing a bio-derived branched primary alcohol having 6 to 12 carbon atoms in the present invention comprise the step of dimerizing the bio-derived linear primary alcohol. The above-mentioned step may consist of only the step of dimerizing the bio-derived linear primary alcohol, or may comprise a purification step or the like using known filtration, centrifugation, oil-water separation, decantation, distillation, deodorization, adsorption, or the like. In addition, when a bio-derived linear primary alcohol having a linear alkenyl group having 3 to 6 carbon atoms is used as the bio-derived linear primary alcohol serving as a raw material, it is only necessary that the above-mentioned step comprise the step of dimerizing the bio-derived linear primary alcohol and the step of hydrogenating the resultant compound. The above-mentioned step may further comprise a purification step or the like using known filtration, centrifugation, oil-water separation, decantation, distillation, deodorization, adsorption, or the like. In addition, when a raw material containing a bio-derived linear aldehyde having a linear alkyl group having 3 to 6 carbon atoms or a bio-derived linear aldehyde having a linear alkenyl group having 3 to 6 carbon atoms is used, it is only necessary that the step of providing a bio-derived branched primary alcohol having 6 to 12 carbon atoms in the present invention comprise the step of dimerizing the bio-derived linear aldehyde and the step of hydrogenating the resultant compound. The above-mentioned step may further comprise a purification step or the like using known filtration, centrifugation, oil-water separation, decantation, distillation, deodorization, adsorption, or the like.

There is no particular limitation on a distillation method that may be used in the step of providing a bio-derived branched primary alcohol having 6 to 12 carbon atoms in the present invention, and a known method may be appropriately selected in accordance with purposes. For example, a method, such as atmospheric distillation, reduced-pressure distillation, molecular distillation, or hydrodistillation, may be used. More specifically, a method, such as single distillation, fractional distillation, flash distillation, steam distillation, vacuum distillation, short-step distillation, thin film distillation, reactive distillation, or extractive distillation, may be used. In addition, there is no particular limitation on a deodorization method that may be used in the step of providing a bio-derived branched primary alcohol having 6 to 12 carbon atoms in the present invention, and for example, a method involving bringing water, water vapor, or an inert gas into contact with a product under ambient pressure or reduced pressure may be used. In addition, there is no particular limitation on an adsorption method that may be used in the step of providing a bio-derived branched primary alcohol having 6 to 12 carbon atoms in the present invention, and for example, a method involving adding an adsorbent, such as activated white clay, activated carbon, magnesium silicate, aluminum silicate, aluminum phosphosilicate, magnesium hydroxide, aluminum hydroxide, magnesium oxide, aluminum oxide, zeolite, or silica gel, stirring the resultant or leaving the resultant to stand still as required, and then removing the adsorbent by a method such as filtration, may be used.

In the present invention, of those described above, from the viewpoint of easily providing a bio-derived ester compound with further reduced odor, as the method of dimerizing the bio-derived linear primary alcohol, a method of dimerizing the bio-derived linear primary alcohols through a reaction for from 10 minutes to 72 hours by heating to from 60°C to 330°C in the presence of a catalyst as required is preferably used, and a method of dimerizing the bio-derived linear primary alcohols, in particular, the same bio-derived linear primary alcohols through a reaction for from 30 minutes to 48 hours by heating to from 120°C to 320°C in the presence of a catalyst is more preferably used.

In addition, in the present invention, of those described above, from the viewpoint of easily providing a bio-derived ester compound with further reduced odor, as the method of dimerizing the bio-derived linear aldehyde, a method of dimerizing the bio-derived linear aldehydes through a reaction for from 10 minutes to 72 hours by heating to from 60°C to 330°C in the presence of a catalyst as required is preferably used, and a method of dimerizing the bio-derived linear aldehydes, in particular, the same bio-derived linear aldehydes through a reaction for from 30 minutes to 48 hours by heating to from 120°C to 320°C in the presence of a catalyst is more preferably used.

In the present invention, by the method described above, bio-derived 2-methylpentanol in the case of using bio-derived n-propanol or bio-derived n-propenyl alcohol as the bio-derived linear primary alcohol, bio-derived 2-ethylhexanol in the case of using bio-derived n-butanol or bio-derived n-butenyl alcohol as the bio-derived linear primary alcohol, bio-derived 2-propylheptanol in the case of using bio-derived n-pentanol or bio-derived n-pentenyl alcohol as the bio-derived linear primary alcohol, and bio-derived 2-butyloctanol in the case of using bio-derived n-hexanol or bio-derived n-hexenyl alcohol as the bio-derived linear primary alcohol may each be obtained as a bio-derived branched primary alcohol having 6 to 12 carbon atoms that can simply produce a bio-derived ester compound with reduced odor. In addition, similarly, by the method described above, bio-derived 2-methylpentanol in the case of using bio-derived n-propionaldehyde or bio-derived n-propene aldehyde as the bio-derived linear aldehyde, bio-derived 2-ethylhexanol in the case of using bio-derived n-butyraldehyde or bio-derived n-propylene aldehyde as the bio-derived linear aldehyde, bio-derived 2-propylheptanol in the case of using bio-derived n-valeraldehyde or bio-derived n-butylene aldehyde as the bio-derived linear aldehyde, and bio-derived 2-butyloctanol in the case of using bio-derived n-hexylaldehyde or bio-derived n-pentene aldehyde as the bio-derived linear aldehyde may each be obtained as a bio-derived branched primary alcohol having 6 to 12 carbon atoms that can simply produce a bio-derived ester compound with reduced odor.

In the present invention, from the viewpoints of the reduction in odor of a bio-derived ester compound to be obtained and various characteristics thereof, the step of providing a bio-derived branched primary alcohol preferably comprises dimerizing the bio-derived linear primary alcohols, in particular, the same bio-derived linear primary alcohols.

The step of providing a bio-derived ester compound in the present invention is the step of providing a bio-derived ester compound by using the bio-derived branched primary alcohol having 6 to 12 carbon atoms obtained in the above-mentioned step and a carboxylic acid-based compound having a hydrocarbon group having 1 to 22 carbon atoms or an anhydride thereof. In this case, specific examples of the bio-derived branched primary alcohol having 6 to 12 carbon atoms obtained in the above-mentioned step include bio-derived 2-methylpentanol, bio-derived 2-ethylhexanol, bio-derived 2-propylheptanol, and bio-derived 2-butyloctanol, and one kind or two or more kinds thereof may be used. In the bio-derived branched primary alcohol having 6 to 12 carbon atoms to be used, it is not required that all of 6 to 12 carbon atoms for forming the bio-derived branched primary alcohol having 6 to 12 carbon atoms be bio-derived. However, from the viewpoints of environmental friendliness, ease of production, and the like, it is preferred that 50% or more of the carbon atoms for forming the bio-derived branched primary alcohol having 6 to 12 carbon atoms be bio-derived carbon atoms, and it is more preferred that all of the carbon atoms for forming the bio-derived branched primary alcohol having 6 to 12 carbon atoms be bio-derived carbon atoms.

Examples of the carboxylic acid-based compound having a hydrocarbon group having 1 to 22 carbon atoms that may be used in the step of providing a bio-derived ester compound in the present invention include an aliphatic monocarboxylic acid having a linear saturated aliphatic hydrocarbon group having 1 to 22 carbon atoms, an aliphatic monocarboxylic acid having a branched saturated aliphatic hydrocarbon group having 4 to 22 carbon atoms, an aliphatic monocarboxylic acid having a linear unsaturated aliphatic hydrocarbon group having 2 to 22 carbon atoms, an aliphatic monocarboxylic acid having a branched unsaturated aliphatic hydrocarbon group having 4 to 22 carbon atoms, an alicyclic monocarboxylic acid having an alicyclic hydrocarbon group having 4 to 22 carbon atoms, and an aromatic monocarboxylic acid having an aromatic hydrocarbon group having 6 to 22 carbon atoms, and one kind or two or more kinds thereof may be used. In addition, examples of the anhydride of the carboxylic acid having a hydrocarbon group having 1 to 22 carbon atoms that may be used in the present invention include anhydrides of the above-mentioned respective carboxylic acids, and one kind or two or more kinds thereof may be used. In the present invention, a carboxylic acid-based compound having a hydroxy group, an aldehyde group, a carbonyl group, an ester bond, an ether bond, or the like in a molecule of each of the above-mentioned carboxylic acids may also be used, and a carboxylic acid-based compound having two or more carboxy groups in a molecule thereof may also be used. In addition, in the present invention, each of a methyl-esterified product and an ethyl-esterified product of the above-mentioned carboxylic acid having a hydrocarbon group having 1 to 22 carbon atoms may be used as the carboxylic acid-based compound having a hydrocarbon group having 1 to 22 carbon atoms because an ester exchange reaction occurs and a bioester compound is obtained when the methyl-esterified product or the ethyl-esterified product is used together with the bio-derived branched primary alcohol.

The aliphatic monocarboxylic acid having a linear saturated aliphatic hydrocarbon group having 1 to 22 carbon atoms may be used without any particular limitation as long as the aliphatic monocarboxylic acid has a linear saturated aliphatic hydrocarbon group having 1 to 22 carbon atoms and one carboxy group in a molecule thereof, and is a compound belonging to acyclic aliphatic compounds defined in the IUPAC Compendium of Chemical Terminology, second edition (1997). Examples thereof include: an aliphatic monocarboxylic acid compound consisting of a linear saturated aliphatic hydrocarbon group having 1 to 22 carbon atoms and one carboxy group; and an aliphatic hydroxymonocarboxylic acid compound consisting of a linear saturated aliphatic hydrocarbon group having 1 to 22 carbon atoms, one carboxy group, and one hydroxy group. Examples of the aliphatic monocarboxylic acid compound consisting of a linear saturated aliphatic hydrocarbon group having 1 to 22 carbon atoms and one carboxy group out of those compounds include acetic acid, propanoic acid, butanoic acid, pentanoic acid, hexanoic acid, heptanoic acid, octanoic acid, nonanoic acid, decanoic acid, undecaonoic acid, dodecanoic acid (lauric acid), tridecanoic acid, tetradecanoic acid (myristic acid), pentadecanoic acid, hexadecanoic acid (palmitic acid), heptadecanoic acid, octadecanoic acid (stearic acid), nonadecanoic acid, icosanoic acid (arachidic acid), eicosanoic acid, henicosanoic acid (behenic acid), and heneicosanoic acid. In addition, examples of the aliphatic hydroxymonocarboxylic acid compound consisting of a linear saturated aliphatic hydrocarbon group having 1 to 22 carbon atoms, one carboxy group, and one hydroxy group include hydroxyacetic acid, 2-hydroxypropanoic acid, 3-hydroxybutanoic acid, 4-hydroxypentanoic acid, 5-hydroxyhexanoic acid, 6-hydroxyheptanoic acid, 7-hydroxyoctanoic acid, 8-hydroxynonanoic acid, 9-hydroxydecanoic acid, 10-hydroxyundecanoic acid, 11-hydroxydodecanoic acid, 12-hydroxytridecanoic acid, 13-hydroxytetradecanoic acid, 14-hydroxypentadecanoic acid, 15-hydroxyhexadecanoic acid, 16-hydroxyheptadecanoic acid, 17-hydroxyoctadecanoic acid, 18-hydroxynonadecanoic acid, 19-hydroxyicosanoic acid, 20-hydroxyeicosanoic acid, 21-hydroxyhenicosanoic acid, 22-hydroxyheneicosanoic acid, and isomers thereof. Of those, in the present invention, from the viewpoint of easily providing a bio-derived ester compound with reduced odor, one kind or two or more kinds selected from the group consisting of: an aliphatic monocarboxylic acid compound consisting of a linear saturated aliphatic hydrocarbon group having 8 to 20 carbon atoms and one carboxy group; and an aliphatic hydroxymonocarboxylic acid compound consisting of a linear saturated aliphatic hydrocarbon group having 8 to 20 carbon atoms, one carboxy group, and one hydroxy group are preferably used, one kind or two or more kinds selected from the group consisting of: an aliphatic monocarboxylic acid compound consisting of a linear saturated aliphatic hydrocarbon group having 10 to 18 carbon atoms and one carboxy group; and an aliphatic hydroxymonocarboxylic acid compound consisting of a linear saturated aliphatic hydrocarbon group having 10 to 18 carbon atoms, one carboxy group, and one hydroxy group are more preferably used, an aliphatic monocarboxylic acid compound consisting of a linear saturated aliphatic hydrocarbon group having 10 to 18 carbon atoms and one carboxy group is still more preferably used, and an aliphatic monocarboxylic acid compound consisting of a linear saturated aliphatic hydrocarbon group having 11 to 17 carbon atoms and one carboxy group is particularly preferably used.

The aliphatic monocarboxylic acid having a branched saturated aliphatic hydrocarbon group having 4 to 22 carbon atoms may be used without any particular limitation as long as the aliphatic monocarboxylic acid has a branched saturated aliphatic hydrocarbon group having 4 to 22 carbon atoms and one carboxy group in a molecule thereof, and is a compound belonging to acyclic aliphatic compounds defined in the IUPAC Compendium of Chemical Terminology, second edition (1997). Examples thereof include: an aliphatic monocarboxylic acid compound consisting of a branched saturated aliphatic hydrocarbon group having 4 to 22 carbon atoms and one carboxy group; and an aliphatic monocarboxylic acid compound consisting of a branched saturated aliphatic hydrocarbon group having 4 to 22 carbon atoms, one carboxy group, and one hydroxy group. Examples of the aliphatic monocarboxylic acid compound consisting of a branched saturated aliphatic hydrocarbon group having 4 to 22 carbon atoms and one carboxy group out of those compounds include isopentanoic acid, isohexanoic acid, 3-methylpentanoic acid, 4-methylpentanoic acid, isoheptanoic acid, isooctanoic acid, 2-ethylhexanoic acid, isononanoic acid, isodecanoic acid, isoundecanoic acid, isododecanoic acid, isotridecanoic acid, isotetradecanoic acid, isopentadecanoic acid, isohexadecanoic acid, isoheptadecanoic acid, isooctadecanoic acid, isononadecanoic acid, isoicosanoic acid, isoeicosanoic acid, isohenicosanoic acid, and isoheneicosanoic acid. In addition, an example of the aliphatic monocarboxylic acid compound consisting of a branched saturated aliphatic hydrocarbon group having 4 to 22 carbon atoms, one carboxy group, and one hydroxy group is an aliphatic monocarboxylic acid compound in which one of hydrogen atoms in the above-mentioned aliphatic monocarboxylic acid compound consisting of a branched saturated aliphatic hydrocarbon group having 4 to 22 carbon atoms and one carboxy group is substituted with a hydroxy group. Of those, in the present invention, from the viewpoint of easily providing a bio-derived ester compound with reduced odor, one kind or two or more kinds selected from the group consisting of: an aliphatic monocarboxylic acid compound consisting of a branched saturated aliphatic hydrocarbon group having 8 to 20 carbon atoms and one carboxy group; and an aliphatic monocarboxylic acid compound consisting of a branched saturated aliphatic hydrocarbon group having 8 to 20 carbon atoms, one carboxy group, and one hydroxy group are preferably used, one kind or two or more kinds selected from the group consisting of: an aliphatic monocarboxylic acid compound consisting of a branched saturated aliphatic hydrocarbon group having 10 to 18 carbon atoms and one carboxy group; and an aliphatic monocarboxylic acid compound consisting of a branched saturated aliphatic hydrocarbon group having 10 to 18 carbon atoms, one carboxy group, and one hydroxy group are more preferably used, an aliphatic monocarboxylic acid compound consisting of a branched saturated aliphatic hydrocarbon group having 10 to 18 carbon atoms and one carboxy group is still more preferably used, and an aliphatic monocarboxylic acid compound consisting of a branched saturated aliphatic hydrocarbon group having 11 to 17 carbon atoms and one carboxy group is particularly preferably used.

The aliphatic monocarboxylic acid having a linear unsaturated aliphatic hydrocarbon group having 2 to 22 carbon atoms may be used without any particular limitation as long as the aliphatic monocarboxylic acid has a linear unsaturated aliphatic hydrocarbon group having 2 to 22 carbon atoms and one carboxy group in a molecule thereof, and is a compound belonging to acyclic aliphatic compounds defined in the IUPAC Compendium of Chemical Terminology, second edition (1997). Examples thereof include: an aliphatic monocarboxylic acid compound consisting of a linear unsaturated aliphatic hydrocarbon group having 2 to 22 carbon atoms and one carboxy group; and an aliphatic monocarboxylic acid compound consisting of a linear unsaturated aliphatic hydrocarbon group having 2 to 22 carbon atoms, one carboxy group, and one hydroxy group. Examples of the aliphatic monocarboxylic acid compound consisting of a linear unsaturated aliphatic hydrocarbon group having 2 to 22 carbon atoms and one carboxy group out of those compounds include propenoic acid, butenoic acid, pentenoic acid, hexenoic acid, heptenoic acid, octenoic acid, nonenoic acid, decenoic acid, undecenoic acid, dodecenoic acid, tridecenoic acid, tetradecenoic acid, pentadecenoic acid, hexadecenoic acid, heptadecenoic acid, octadecenoic acid, nonadecenoic acid, icosenoic acid, eicosenoic acid, henicosenoic acid, heneicosenoic acid, and isomers thereof. More specific examples thereof include 2-propenoic acid, 3-butenoic acid, 4-pentenoic acid, 5-hexenoic acid, 6-heptenoic acid, 7-octenoic acid, 8-nonenoic acid, 9-decenoic acid, 10-undecenoic acid, 11-dodecenoic acid, 12-tridecenoic acid, 13-tetradecenoic acid, 14-pentadecenoic acid, palmitooleic acid, 15-hexadecenoic acid, 16-heptadecenoic acid, oleic acid, elaidic acid, vaccenic acid, linoleic acid, α-linolenic acid, β-linolenic acid, 17-octadecenoic acid, 18-nonadecenoic acid, arachidonic acid, eicosapentaenoic acid, 19-icosenoic acid, 20-eicosenoic acid, 21-henicosenoic acid, erucic acid, docosahexaenoic acid, and 22-heneicosenoic acid. In addition, an example of the aliphatic monocarboxylic acid compound consisting of a linear unsaturated aliphatic hydrocarbon group having 2 to 22 carbon atoms, one carboxy group, and one hydroxy group is an aliphatic monocarboxylic acid compound in which one of hydrogen atoms in the above-mentioned aliphatic monocarboxylic acid compound consisting of a linear unsaturated aliphatic hydrocarbon group having 2 to 22 carbon atoms and one carboxy group is substituted with a hydroxy group. Of those, in the present invention, from the viewpoint of easily providing a bio-derived ester compound with reduced odor, one kind or two or more kinds selected from the group consisting of: an aliphatic monocarboxylic acid compound consisting of a linear unsaturated aliphatic hydrocarbon group having 8 to 20 carbon atoms and one carboxy group; and an aliphatic hydroxymonocarboxylic acid compound consisting of a linear unsaturated aliphatic hydrocarbon group having 8 to 20 carbon atoms, one carboxy group, and one hydroxy group are preferably used, one kind or two or more kinds selected from the group consisting of: an aliphatic monocarboxylic acid compound consisting of a linear unsaturated aliphatic hydrocarbon group having 10 to 18 carbon atoms and one carboxy group; and an aliphatic hydroxymonocarboxylic acid compound consisting of a linear unsaturated aliphatic hydrocarbon group having 10 to 18 carbon atoms, one carboxy group, and one hydroxy group are more preferably used, an aliphatic monocarboxylic acid compound consisting of a linear unsaturated aliphatic hydrocarbon group having 10 to 18 carbon atoms and one carboxy group is still more preferably used, and an aliphatic monocarboxylic acid compound consisting of a linear unsaturated aliphatic hydrocarbon group having 11 to 17 carbon atoms and one carboxy group is particularly preferably used.

The aliphatic monocarboxylic acid having a branched unsaturated aliphatic hydrocarbon group having 4 to 22 carbon atoms may be used without any particular limitation as long as the aliphatic monocarboxylic acid has a branched unsaturated aliphatic hydrocarbon group having 4 to 22 carbon atoms and one carboxy group in a molecule thereof, and is a compound belonging to acyclic aliphatic compounds defined in the IUPAC Compendium of Chemical Terminology, second edition (1997). Examples thereof include: an aliphatic monocarboxylic acid compound consisting of a branched unsaturated aliphatic hydrocarbon group having 4 to 22 carbon atoms and one carboxy group; and an aliphatic monocarboxylic acid compound consisting of a branched unsaturated aliphatic hydrocarbon group having 4 to 22 carbon atoms, one carboxy group, and one hydroxy group. Examples of the aliphatic monocarboxylic acid compound consisting of a branched unsaturated aliphatic hydrocarbon group having 4 to 22 carbon atoms and one carboxy group out of those compounds include isopentenoic acid, isohexenoic acid, isoheptenoic acid, isooctenoic acid, isononenoic acid, isodecenoic acid, isoundecenoic acid, isododecenoic acid, isotridecenoic acid, isotetradecenoic acid, isopentadecenoic acid, isohexadecenoic acid, isoheptadecenoic acid, isooctadecenoic acid, isononadecenoic acid, isoicosenoic acid, isoeicosenoic acid, isohenicosenoic acid, isoheneicosenoic acid, and isomers thereof. In addition, an example of the aliphatic monocarboxylic acid compound consisting of a branched unsaturated aliphatic hydrocarbon group having 4 to 22 carbon atoms, one carboxy group, and one hydroxy group is an aliphatic monocarboxylic acid compound in which one of hydrogen atoms in the above-mentioned aliphatic monocarboxylic acid compound consisting of a branched unsaturated aliphatic hydrocarbon group having 4 to 22 carbon atoms and one carboxy group is substituted with a hydroxy group. Of those, in the present invention, from the viewpoint of easily providing a bio-derived ester compound with reduced odor, one kind or two or more kinds selected from the group consisting of: an aliphatic monocarboxylic acid compound consisting of a branched unsaturated aliphatic hydrocarbon group having 8 to 20 carbon atoms and one carboxy group; and an aliphatic monocarboxylic acid compound consisting of a branched unsaturated aliphatic hydrocarbon group having 8 to 20 carbon atoms, one carboxy group, and one hydroxy group are preferably used, one kind or two or more kinds selected from the group consisting of: an aliphatic monocarboxylic acid compound consisting of a branched unsaturated aliphatic hydrocarbon group having 10 to 18 carbon atoms and one carboxy group; and an aliphatic monocarboxylic acid compound consisting of a branched unsaturated aliphatic hydrocarbon group having 10 to 18 carbon atoms, one carboxy group, and one hydroxy group are more preferably used, an aliphatic monocarboxylic acid compound consisting of a branched unsaturated aliphatic hydrocarbon group having 10 to 18 carbon atoms and one carboxy group is still more preferably used, and an aliphatic monocarboxylic acid compound consisting of a branched unsaturated aliphatic hydrocarbon group having 11 to 17 carbon atoms and one carboxy group is particularly preferably used.

The alicyclic monocarboxylic acid having an alicyclic hydrocarbon group having 4 to 22 carbon atoms may be used without any particular limitation as long as the alicyclic monocarboxylic acid has an alicyclic hydrocarbon group having 4 to 22 carbon atoms and one carboxy group in a molecule thereof, and is a compound belonging to alicyclic compounds defined in the IUPAC Compendium of Chemical Terminology, second edition (1997). Examples thereof include: an alicyclic monocarboxylic acid compound consisting of an alicyclic hydrocarbon group having 4 to 22 carbon atoms and one carboxy group; and an alicyclic monocarboxylic acid compound consisting of an alicyclic hydrocarbon group having 4 to 22 carbon atoms, one carboxy group, and one hydroxy group. Examples of the alicyclic monocarboxylic acid compound consisting of an alicyclic hydrocarbon group having 4 to 22 carbon atoms and one carboxy group out of those compounds include methylcyclopropanecarboxylic acid, ethylcyclopropanecarboxylic acid, dimethylcyclopropanecarboxylic acid, propylcyclopropanecarboxylic acid, diethylcyclopropanecarboxylic acid, butylcyclopropanecarboxylic acid, t-butylcyclopropanecarboxylic acid, pentylcyclopropanecarboxylic acid, hexylcyclopropanecarboxylic acid, heptylcyclopropanecarboxylic acid, octylcyclopropanecarboxylic acid, nonylcyclopropanecarboxylic acid, decylcyclopropanecarboxylic acid, undecylcyclopropanecarboxylic acid, dodecylcyclopropanecarboxylic acid, tridecylcyclopropanecarboxylic acid, tetradecylcyclopropanecarboxylic acid, pentadecylcyclopropanecarboxylic acid, hexadecylcyclopropanecarboxylic acid, heptadecylcyclopropanecarboxylic acid, octadecylcyclopropanecarboxylic acid, nonadecylcyclopropanecarboxylic acid, cyclobutanecarboxylic acid, cyclopentanecarboxylic acid, cyclohexanecarboxylic acid, methylcyclohexanecarboxylic acid, ethylcyclohexanecarboxylic acid, dimethylcyclohexanecarboxylic acid, and propylcyclohexanecarboxylic acid. In addition, an example of the alicyclic monocarboxylic acid compound consisting of an alicyclic hydrocarbon group having 4 to 22 carbon atoms, one carboxy group, and one hydroxy group is an alicyclic monocarboxylic acid compound in which one of hydrogen atoms in the above-mentioned alicyclic monocarboxylic acid compound consisting of an alicyclic hydrocarbon group having 4 to 22 carbon atoms and one carboxy group is substituted with a hydroxy group. Of those, in the present invention, from the viewpoint of easily providing a bio-derived ester compound with reduced odor, one kind or two or more kinds selected from the group consisting of: an alicyclic monocarboxylic acid compound consisting of an alicyclic hydrocarbon group having 4 to 22 carbon atoms and one carboxy group; and an alicyclic monocarboxylic acid compound consisting of an alicyclic hydrocarbon group having 4 to 22 carbon atoms, one carboxy group, and one hydroxy group are preferably used, one kind or two or more kinds selected from the group consisting of: an alicyclic monocarboxylic acid compound consisting of an alicyclic hydrocarbon group having 6 to 18 carbon atoms and one carboxy group; and an alicyclic monocarboxylic acid compound consisting of an alicyclic hydrocarbon group having 6 to 18 carbon atoms, one carboxy group, and one hydroxy group are more preferably used, and an alicyclic monocarboxylic acid compound consisting of an alicyclic hydrocarbon group having 6 to 16 carbon atoms and one carboxy group is still more preferably used.

The aromatic monocarboxylic acid having an aromatic hydrocarbon group having 6 to 22 carbon atoms may be used without any particular limitation as long as the aromatic monocarboxylic acid has an aromatic hydrocarbon group having 6 to 22 carbon atoms and one carboxy group in a molecule thereof, and is a compound belonging to aromatic compounds defined in the IUPAC Compendium of Chemical Terminology, second edition (1997). Examples thereof include: an aromatic monocarboxylic acid compound consisting of an aromatic hydrocarbon group having 6 to 22 carbon atoms and one carboxy group; and an aromatic monocarboxylic acid compound consisting of an aromatic hydrocarbon group having 6 to 22 carbon atoms, one carboxy group, and one hydroxy group. Examples of such aromatic monocarboxylic acid compound include benzoic acid, o-oxybenzoic acid (salicylic acid), p-oxybenzoic acid, m-oxybenzoic acid, o-toluic acid, m-toluic acid, p-toluic acid, 3-hydroxy-o-toluic acid, 3-hydroxy-m-toluic acid, 3-hydroxy-p-toluic acid, o-ethylbenzoic acid, methylbenzoic acid, p-ethylbenzoic acid, p-hydroxyethylbenzoic acid, o-propylbenzoic acid, m-propylbenzoic acid, p-propylbenzoic acid, p-hydroxypropylbenzoic acid, o-butylbenzoic acid, m-butylbenzoic acid, p-butylbenzoic acid, p-hydroxybutylbenzoic acid, p-hexylbenzoic acid, p-hydroxyhexylbenzoic acid, p-heptylbenzoic acid, p-hydroxyheptylbenzoic acid, p-octylbenzoic acid, p-hydroxyoctylbenzoic acid, p-ethylhexylbenzoic acid, p-nonylbenzoic acid, p-hydroxynonylbenzoic acid, p-decylbenzoic acid, p-hydroxydecylbenzoic acid, p-undecylbenzoic acid, p-hydroxyundecylbenzoic acid, p-dodecylbenzoic acid, p-hydroxydodecylbenzoic acid, p-tridecylbenzoic acid, p-hydroxytridecylbenzoic acid, p-tetradecylbenzoic acid, p-hydroxytetradecylbenzoic acid, p-pentadecylbenzoic acid, p-hydroxypentadecylbenzoic acid, p-hexadecylbenzoic acid, and p-hydroxyhexadecylbenzoic acid. Of those, in the present invention, from the viewpoint of easily providing a bio-derived ester compound with reduced odor, one kind or two or more kinds selected from the group consisting of: an aromatic monocarboxylic acid compound consisting of an aromatic hydrocarbon group having 6 to 22 carbon atoms and one carboxy group; and an aromatic monocarboxylic acid compound consisting of an aromatic hydrocarbon group having 6 to 22 carbon atoms, one carboxy group, and one hydroxy group are preferably used, one kind or two or more kinds selected from the group consisting of: an aromatic monocarboxylic acid compound consisting of an aromatic hydrocarbon group having 6 to 18 carbon atoms and one carboxy group; and an aromatic monocarboxylic acid compound consisting of an aromatic hydrocarbon group having 6 to 18 carbon atoms, one carboxy group, and one hydroxy group are more preferably used, one kind or two or more kinds selected from the group consisting of: an aromatic monocarboxylic acid compound consisting of an aromatic hydrocarbon group having 6 to 10 carbon atoms and one carboxy group; and an aromatic monocarboxylic acid compound consisting of an aromatic hydrocarbon group having 6 to 10 carbon atoms, one carboxy group, and one hydroxy group are still more preferably used, and an aromatic monocarboxylic acid compound consisting of an aromatic hydrocarbon group having 6 to 10 carbon atoms, one carboxy group, and one hydroxy group is particularly preferably used.

In the present invention, of those, from the viewpoints of the reduction in odor of a bio-derived ester compound to be obtained and various characteristics thereof, as the carboxylic acid-based compound having a hydrocarbon group having 1 to 22 carbon atoms or the anhydride thereof, one kind or two or more kinds selected from the group consisting of: an aliphatic monocarboxylic acid compound having a linear saturated aliphatic hydrocarbon group having 1 to 22 carbon atoms and one carboxy group, and an anhydride thereof; an aliphatic monocarboxylic acid compound consisting of a branched saturated aliphatic hydrocarbon group having 4 to 22 carbon atoms and one carboxy group, and an anhydride thereof; an aliphatic monocarboxylic acid compound consisting of a linear unsaturated aliphatic hydrocarbon group having 2 to 22 carbon atoms and one carboxy group, and an anhydride thereof; an aliphatic monocarboxylic acid compound consisting of a branched unsaturated aliphatic hydrocarbon group having 4 to 22 carbon atoms and one carboxy group, and an anhydride thereof; an alicyclic monocarboxylic acid compound consisting of an alicyclic hydrocarbon group having 4 to 22 carbon atoms and one carboxy group, and an anhydride thereof; and an aromatic monocarboxylic acid compound consisting of an aromatic hydrocarbon group having 6 to 22 carbon atoms, one carboxy group, and one hydroxy group, and an anhydride thereof are preferably used, one kind or two or more kinds selected from the group consisting of: an aliphatic monocarboxylic acid compound consisting of a linear saturated aliphatic hydrocarbon group having 1 to 22 carbon atoms and one carboxy group, and an anhydride thereof; and an aromatic monocarboxylic acid compound consisting of an aromatic hydrocarbon group having 6 to 22 carbon atoms, one carboxy group, and one hydroxy group are more preferably used, one kind or two or more kinds selected from the group consisting of: an aliphatic monocarboxylic acid compound consisting of a linear saturated aliphatic hydrocarbon group having 8 to 20 carbon atoms and one carboxy group, and an anhydride thereof; and an aromatic monocarboxylic acid compound consisting of an aromatic hydrocarbon group having 6 to 16 carbon atoms, one carboxy group, and one hydroxy group are still more preferably used, one kind or two or more kinds selected from the group consisting of: an aliphatic monocarboxylic acid compound consisting of a linear saturated aliphatic hydrocarbon group having 10 to 18 carbon atoms and one carboxy group; and an aromatic monocarboxylic acid compound consisting of an aromatic hydrocarbon group having 6 to 12 carbon atoms, one carboxy group, and one hydroxy group are still more preferably used, one kind or two or more kinds selected from the group consisting of: an aliphatic monocarboxylic acid compound consisting of a linear saturated aliphatic hydrocarbon group having 11 to 17 carbon atoms and one carboxy group; and an aromatic monocarboxylic acid compound consisting of an aromatic hydrocarbon group having 6 to 10 carbon atoms, one carboxy group, and one hydroxy group are still more preferably used, and one kind or two or more kinds selected from the group consisting of: lauric acid; myristic acid; palmitic acid; stearic acid; and salicylic acid are particularly preferably used. The carboxylic acid-based compound having a hydrocarbon group having 1 to 22 carbon atoms to be used in the present invention or the anhydride thereof may be a compound derived from a petroleum raw material or a bio-derived compound obtained from a plant resource or the like. However, from the viewpoints of environmental friendliness and the like, it is preferred that 50% or more of carbon atoms for forming the carboxylic acid-based compound having a hydrocarbon group having 1 to 22 carbon atoms or the anhydride thereof be bio-derived carbon atoms, and it is more preferred that all of the carbon atoms be bio-derived carbon atoms. As a method of producing an ester compound, a method of providing an ester compound with reduced odor by using the bio-derived branched primary alcohol having 6 to 12 carbon atoms derived from a petroleum raw material produced by an oxo method from propylene or the like derived from a petroleum raw material and the bio-derived carboxylic acid-based compound having a hydrocarbon group having 1 to 22 carbon atoms or the anhydride thereof is assumed, but as in Comparative Examples described later, a bio-derived ester compound with reduced odor cannot be obtained by this method.

In the present invention, there is no particular limitation on the method of providing a bio-derived ester compound by using the bio-derived branched primary alcohol having 6 to 12 carbon atoms and the carboxylic acid-based compound having a hydrocarbon group having 1 to 22 carbon atoms or the anhydride thereof, and a known method may be used. An example thereof is a method involving causing the bio-derived branched primary alcohol having 6 to 12 carbon atoms and the carboxylic acid-based compound having a hydrocarbon group having 1 to 22 carbon atoms or the anhydride thereof to react with each other by a known esterification reaction. A specific example of such method is a method involving mixing the bio-derived branched primary alcohol having 6 to 12 carbon atoms and the carboxylic acid-based compound having a hydrocarbon group having 1 to 22 carbon atoms or the anhydride thereof in the presence of a solvent, a catalyst, or the like as required, and causing the bio-derived branched primary alcohol and the carboxylic acid-based compound or the anhydride thereof to react with each other at room temperature or by heating under reduced pressure , under ambient pressure, or under increased pressure.

There is no particular limitation on the solvent that may be used in the esterification reaction between the bio-derived branched primary alcohol having 6 to 12 carbon atoms and the carboxylic acid-based compound having a hydrocarbon group having 1 to 22 carbon atoms or the anhydride thereof as long as the solvent is a known solvent that may be used in the esterification reaction. Examples thereof include hexane, heptane, octane, cyclohexane, benzene, toluene, xylene, ethylbenzene, chloroform, dichloromethane, 1,2-dichloroethane, diethyl ether, dimethoxyethane, tetrahydrofuran, dioxane, acetone, and methyl ethyl ketone, and one kind or two or more kinds thereof may be used.

In the present invention, from the viewpoint of simply performing the esterification reaction between the bio-derived branched primary alcohol having 6 to 12 carbon atoms and the carboxylic acid-based compound having a hydrocarbon group having 1 to 22 carbon atoms or the anhydride thereof, it is preferred that the method of providing a bio-derived ester compound by using the bio-derived branched primary alcohol having 6 to 12 carbon atoms and the carboxylic acid-based compound having a hydrocarbon group having 1 to 22 carbon atoms or the anhydride thereof be a method involving subjecting the bio-derived branched primary alcohol having 6 to 12 carbon atoms and the carboxylic acid-based compound having a hydrocarbon group having 1 to 22 carbon atoms or the anhydride thereof to the esterification reaction in the absence of a solvent.

When the solvent is used in the esterification reaction between the bio-derived branched primary alcohol having 6 to 12 carbon atoms and the carboxylic acid-based compound having a hydrocarbon group having 1 to 22 carbon atoms or the anhydride thereof, there is no particular limitation on the amount of the solvent, and the amount of the solvent may be appropriately adjusted in accordance with purposes. From the viewpoint of efficiently performing the esterification reaction, the usage amount of the solvent is set to preferably from 1 part by mass to 500 parts by mass, more preferably from 10 parts by mass to 300 parts by mass, still more preferably from 3 parts by mass to 200 parts by mass when the total amount of the bio-derived branched primary alcohol having 6 to 12 carbon atoms and the carboxylic acid-based compound having a hydrocarbon group having 1 to 22 carbon atoms or the anhydride thereof to be used is set to 100 parts by mass.

There is no particular limitation on the catalyst that may be used in the esterification reaction between the bio-derived branched primary alcohol having 6 to 12 carbon atoms and the carboxylic acid-based compound having a hydrocarbon group having 1 to 22 carbon atoms or the anhydride thereof as long as the catalyst is a known catalyst that may be used in the esterification reaction. Examples thereof include hydrochloric acid, sulfuric acid, nitric acid, phosphoric acid, perchloric acid, trifluoroacetic acid, methanesulfonic acid, ethanesulfonic acid, propanesulfonic acid, butanesulfonic acid, p-toluenesulfonic acid, trifluoromethanesulfonic acid, dodecylbenzenesulfonic acid, naphthalenesulfonic acid, methylphosphonic acid, phenylphosphonic acid, aluminum lactate, lithium fluoride, potassium chloride, cesium chloride, calcium chloride, iron chloride, aluminum phosphate, zinc acetate, tetrapropyl titanate, tetrabutyl titanate, dibutyltin oxide, dioctyltin oxide, tetrapropyl zirconium, tetrabutyl zirconium, zirconium n-propylate, zirconium octylate, zirconium stearate, oxozirconium n-propylate, oxozirconium octylate, oxozirconium stearate, zirconium tetraacetylacetonate, zirconium chloride, a cation exchange resin, and an anion exchange resin, and one kind or two or more kinds thereof may be used. Of those, from the viewpoint of providing a bio-derived ester compound with further reduced odor, as the catalyst, one kind or two or more kinds selected from the group consisting of: hydrochloric acid; sulfuric acid; nitric acid; phosphoric acid; perchloric acid; methanesulfonic acid; p-toluenesulfonic acid; tetrapropyl titanate; zirconium octylate; and oxozirconium octylate are preferably used, and one kind or two or more kinds selected from the group consisting of: hydrochloric acid; sulfuric acid; phosphoric acid; p-toluenesulfonic acid; and zirconium octylate are more preferably used.

When the catalyst is used in the esterification reaction between the bio-derived branched primary alcohol having 6 to 12 carbon atoms and the carboxylic acid-based compound having a hydrocarbon group having 1 to 22 carbon atoms or the anhydride thereof, there is no particular limitation on the amount of each catalyst, and the amount of the catalyst may be appropriately adjusted in accordance with purposes. From the viewpoint of efficiently performing the esterification reaction, the usage amount of the catalyst is set to preferably from 0.001 part by mass to 20 parts by mass, more preferably from 0.01 part by mass to 10 parts by mass, still more preferably from 0.1 part by mass to 5 parts by mass, particularly preferably from 0.2 part by mass to 3 parts by mass when the total amount of the bio-derived branched primary alcohol having 6 to 12 carbon atoms and the carboxylic acid-based compound having a hydrocarbon group having 1 to 22 carbon atoms or the anhydride thereof to be used is set to 100 parts by mass.

In the present invention, from the viewpoint of efficiently performing the esterification reaction between the bio-derived branched primary alcohol having 6 to 12 carbon atoms and the carboxylic acid-based compound having a hydrocarbon group having 1 to 22 carbon atoms or the anhydride thereof, it is preferred that the method of providing a bio-derived ester compound by using the bio-derived branched primary alcohol having 6 to 12 carbon atoms and the carboxylic acid-based compound having a hydrocarbon group having 1 to 22 carbon atoms or the anhydride thereof be a method involving subjecting the bio-derived branched primary alcohol having 6 to 12 carbon atoms and the carboxylic acid-based compound having a hydrocarbon group having 1 to 22 carbon atoms or the anhydride thereof to the esterification reaction in the presence of a catalyst.

There is no particular limitation on the temperature when the bio-derived branched primary alcohol having 6 to 12 carbon atoms and the carboxylic acid-based compound having a hydrocarbon group having 1 to 22 carbon atoms or the anhydride thereof are caused to react with each other, and the temperature may be appropriately adjusted in accordance with purposes. The temperature may be set to, for example, from room temperature to 300°C. Of those, from the viewpoint of providing a bio-derived ester compound with further reduced odor, the temperature when the bio-derived branched primary alcohol having 6 to 12 carbon atoms and the carboxylic acid-based compound having a hydrocarbon group having 1 to 22 carbon atoms or the anhydride thereof are caused to react with each other is set to preferably from 50°C to 260°C, more preferably from 60°C to 220°C, still more preferably from 70°C to 200°C, particularly preferably from 80°C to 160°C.

In the present invention, from the viewpoint of efficiently performing the esterification reaction between the bio-derived branched primary alcohol having 6 to 12 carbon atoms and the carboxylic acid-based compound having a hydrocarbon group having 1 to 22 carbon atoms or the anhydride thereof, it is preferred that the method of providing a bio-derived ester compound by using the bio-derived branched primary alcohol having 6 to 12 carbon atoms and the carboxylic acid-based compound having a hydrocarbon group having 1 to 22 carbon atoms or the anhydride thereof be a method involving subjecting the bio-derived branched primary alcohol having 6 to 12 carbon atoms and the carboxylic acid-based compound having a hydrocarbon group having 1 to 22 carbon atoms or the anhydride thereof to the esterification reaction under heated conditions.

There is no particular limitation on the reaction time when the bio-derived branched primary alcohol having 6 to 12 carbon atoms and the carboxylic acid-based compound having a hydrocarbon group having 1 to 22 carbon atoms or the anhydride thereof are caused to react with each other, and the reaction time may be appropriately adjusted in accordance with purposes. The reaction time may be set to, for example, from 10 minutes to 72 hours. Of those, from the viewpoint of providing a bio-derived ester compound with further reduced odor, the reaction time when the bio-derived branched primary alcohol having 6 to 12 carbon atoms and the carboxylic acid-based compound having a hydrocarbon group having 1 to 22 carbon atoms or the anhydride thereof are caused to react with each other is set to preferably from 1 hour to 48 hours, more preferably from 2 hours to 24 hours.

In the present invention, when the esterification reaction between the bio-derived branched primary alcohol having 6 to 12 carbon atoms and the carboxylic acid-based compound having a hydrocarbon group having 1 to 22 carbon atoms or the anhydride thereof is performed under reduced pressure , there is no particular limitation on the pressure thereof, and the pressure may be appropriately adjusted in accordance with purposes. The esterification reaction may be performed, for example, under a pressure of from 0.01 Pa to 80,000 Pa. In addition, when the esterification reaction between the bio-derived branched primary alcohol having 6 to 12 carbon atoms and the carboxylic acid-based compound having a hydrocarbon group having 1 to 22 carbon atoms or the anhydride thereof is performed under pressurized conditions, there is no particular limitation on the pressure thereof, and the pressure may be appropriately adjusted in accordance with purposes. The esterification reaction may be performed, for example, under pressurized conditions of from 0.001 MPa to 10 MPa with respect to the atmospheric pressure.

In the present invention, from the viewpoint of simply performing the esterification reaction between the bio-derived branched primary alcohol having 6 to 12 carbon atoms and the carboxylic acid-based compound having a hydrocarbon group having 1 to 22 carbon atoms or the anhydride thereof, it is preferred that the method of providing a bio-derived ester compound by using the bio-derived branched primary alcohol having 6 to 12 carbon atoms and the carboxylic acid-based compound having a hydrocarbon group having 1 to 22 carbon atoms or the anhydride thereof be a method involving subjecting the bio-derived branched primary alcohol having 6 to 12 carbon atoms and the carboxylic acid-based compound having a hydrocarbon group having 1 to 22 carbon atoms or the anhydride thereof to the esterification reaction under a ambient pressure .

In the present invention, from the viewpoint of simply and efficiently performing the esterification reaction between the bio-derived branched primary alcohol having 6 to 12 carbon atoms and the carboxylic acid-based compound having a hydrocarbon group having 1 to 22 carbon atoms or the anhydride thereof, the method of providing a bio-derived ester compound by using the bio-derived branched primary alcohol having 6 to 12 carbon atoms and the carboxylic acid-based compound having a hydrocarbon group having 1 to 22 carbon atoms or the anhydride thereof is more preferably a method involving subjecting the bio-derived branched primary alcohol having 6 to 12 carbon atoms and the carboxylic acid-based compound having a hydrocarbon group having 1 to 22 carbon atoms or the anhydride thereof to the esterification reaction in the presence of a catalyst and in the absence of a solvent.

In the present invention, from the viewpoint of simply and efficiently performing the esterification reaction between the bio-derived branched primary alcohol having 6 to 12 carbon atoms and the carboxylic acid-based compound having a hydrocarbon group having 1 to 22 carbon atoms or the anhydride thereof, the method of providing a bio-derived ester compound by using the bio-derived branched primary alcohol having 6 to 12 carbon atoms and the carboxylic acid-based compound having a hydrocarbon group having 1 to 22 carbon atoms or the anhydride thereof is more preferably a method involving subjecting the bio-derived branched primary alcohol having 6 to 12 carbon atoms and the carboxylic acid-based compound having a hydrocarbon group having 1 to 22 carbon atoms or the anhydride thereof to the esterification reaction in the presence of a catalyst and under ambient pressure.

In the present invention, from the viewpoint of efficiently performing the esterification reaction between the bio-derived branched primary alcohol having 6 to 12 carbon atoms and the carboxylic acid-based compound having a hydrocarbon group having 1 to 22 carbon atoms or the anhydride thereof, the method of providing a bio-derived ester compound by using the bio-derived branched primary alcohol having 6 to 12 carbon atoms and the carboxylic acid-based compound having a hydrocarbon group having 1 to 22 carbon atoms or the anhydride thereof is more preferably a method involving subjecting the bio-derived branched primary alcohol having 6 to 12 carbon atoms and the carboxylic acid-based compound having a hydrocarbon group having 1 to 22 carbon atoms or the anhydride thereof to the esterification reaction in the presence of a catalyst and under heated conditions.

In addition, from the viewpoint of simply and more efficiently performing the esterification reaction between the bio-derived branched primary alcohol having 6 to 12 carbon atoms and the carboxylic acid-based compound having a hydrocarbon group having 1 to 22 carbon atoms or the anhydride thereof, the method of providing a bio-derived ester compound by using the bio-derived branched primary alcohol having 6 to 12 carbon atoms and the carboxylic acid-based compound having a hydrocarbon group having 1 to 22 carbon atoms or the anhydride thereof is still more preferably a method involving subjecting the bio-derived branched primary alcohol having 6 to 12 carbon atoms and the carboxylic acid-based compound having a hydrocarbon group having 1 to 22 carbon atoms or the anhydride thereof to the esterification reaction in the presence of a catalyst, under heated conditions, and in the absence of a solvent.

In addition, from the viewpoint of simply and more efficiently performing the esterification reaction between the bio-derived branched primary alcohol having 6 to 12 carbon atoms and the carboxylic acid-based compound having a hydrocarbon group having 1 to 22 carbon atoms or the anhydride thereof, the method of providing a bio-derived ester compound by using the bio-derived branched primary alcohol having 6 to 12 carbon atoms and the carboxylic acid-based compound having a hydrocarbon group having 1 to 22 carbon atoms or the anhydride thereof is still more preferably a method involving subjecting the bio-derived branched primary alcohol having 6 to 12 carbon atoms and the carboxylic acid-based compound having a hydrocarbon group having 1 to 22 carbon atoms or the anhydride thereof to the esterification reaction in the presence of a catalyst, under heated conditions, and under ambient pressure.

Of those, in the present invention, from the viewpoint of simply and more efficiently performing the esterification reaction between the bio-derived branched primary alcohol having 6 to 12 carbon atoms and the carboxylic acid-based compound having a hydrocarbon group having 1 to 22 carbon atoms or the anhydride thereof, the method of providing a bio-derived ester compound by using the bio-derived branched primary alcohol having 6 to 12 carbon atoms and the carboxylic acid-based compound having a hydrocarbon group having 1 to 22 carbon atoms or the anhydride thereof is particularly preferably a method involving subjecting the bio-derived branched primary alcohol having 6 to 12 carbon atoms and the carboxylic acid-based compound having a hydrocarbon group having 1 to 22 carbon atoms or the anhydride thereof to the esterification reaction in the presence of a catalyst, under heated conditions, in the absence of a solvent, and under ambient pressure.

In the present invention, of those described above, from the viewpoint of providing a bio-derived ester compound with further reduced odor, as the method of causing the bio-derived branched primary alcohol having 6 to 12 carbon atoms and the carboxylic acid-based compound having a hydrocarbon group having 1 to 22 carbon atoms or the anhydride thereof to react with each other, a method of causing the bio-derived branched primary alcohol having 6 to 12 carbon atoms and the carboxylic acid-based compound having a hydrocarbon group having 1 to 22 carbon atoms or the anhydride thereof to react with each other through a reaction for from 1 hour to 48 hours by heating to from 60°C to 300°C in the presence of a catalyst as required is preferably used, and a method of causing the bio-derived branched primary alcohol having 6 to 12 carbon atoms and the carboxylic acid-based compound having a hydrocarbon group having 1 to 22 carbon atoms or the anhydride thereof to react with each other through a reaction for from 2 hours to 24 hours by heating to from 80°C to 220°C in the presence of a catalyst is more preferably used.

In the esterification reaction between the bio-derived branched primary alcohol having 6 to 12 carbon atoms and the carboxylic acid-based compound having a hydrocarbon group having 1 to 22 carbon atoms or the anhydride thereof, there is no particular limitation on the ratio between the bio-derived branched primary alcohol having 6 to 12 carbon atoms and the carboxylic acid-based compound having a hydrocarbon group having 1 to 22 carbon atoms or the anhydride thereof to be used. However, from the viewpoint of providing a bio-derived ester compound with reduced odor in high yield, for example, the bio-derived branched primary alcohol having 6 to 12 carbon atoms and the carboxylic acid-based compound having a hydrocarbon group having 1 to 22 carbon atoms or the anhydride thereof are used preferably at a molar ratio of from 20:1 to 1:10, more preferably at a molar ratio of from 10:1 to 1:5, still more preferably at a molar ratio of from 5:1 to 1:2, particularly preferably at a molar ratio of from 3:1 to 1:1. In this case, the entire amount of the bio-derived branched primary alcohol having 6 to 12 carbon atoms and the carboxylic acid-based compound having a hydrocarbon group having 1 to 22 carbon atoms or the anhydride thereof to be used may be mixed at a time to react with each other, or the bio-derived branched primary alcohol having 6 to 12 carbon atoms and/or the carboxylic acid-based compound having a hydrocarbon group having 1 to 22 carbon atoms or the anhydride thereof may be each divided into portions multiple times to react with each other.

The esterification reaction between the bio-derived branched primary alcohol having 6 to 12 carbon atoms and the carboxylic acid-based compound having a hydrocarbon group having 1 to 22 carbon atoms or the anhydride thereof is an equilibrium reaction, and hence it is impossible in actuality to esterify the entire amount of the raw materials to be used. Thus, in the esterification reaction between the bio-derived branched primary alcohol having 6 to 12 carbon atoms and the carboxylic acid-based compound having a hydrocarbon group having 1 to 22 carbon atoms or the anhydride thereof, although depending on the kinds and amounts of the raw materials to be used and the reaction conditions, the reaction may be terminated when the esterification rate calculated based on the amounts of the bio-derived branched primary alcohol having 6 to 12 carbon atoms and the carboxylic acid-based compound having a hydrocarbon group having 1 to 22 carbon atoms or the anhydride thereof reaches a desired range.

It is only necessary that the step of providing a bio-derived ester compound in the present invention comprise the step of providing a bio-derived ester compound by using the bio-derived branched primary alcohol having 6 to 12 carbon atoms and the carboxylic acid-based compound having a hydrocarbon group having 1 to 22 carbon atoms or the anhydride thereof. The above-mentioned step may consist of only the step of providing a bio-derived ester compound by causing the bio-derived branched primary alcohol having 6 to 12 carbon atoms and the carboxylic acid-based compound having a hydrocarbon group having 1 to 22 carbon atoms or the anhydride thereof to react with each other, or may comprise a purification step of a product or the like using a known method, such as filtration, centrifugation, oil-water separation, decantation, distillation, deodorization, adsorption, as required.

There is no particular limitation on a distillation method that may be used in the step of providing a bio-derived ester compound in the present invention, and the distillation method may be appropriately selected in accordance with purposes. For example, a method, such as atmospheric distillation, reduced-pressure distillation, molecular distillation, or hydrodistillation, may be used. More specifically, a method, such as single distillation, fractional distillation, flash distillation, steam distillation, vacuum distillation, short-step distillation, thin film distillation, reactive distillation, or extractive distillation, may be used. In addition, there is also no particular limitation on a deodorization method that may be used in the step of providing a bio-derived ester compound in the present invention, and the deodorization method may be appropriately selected in accordance with purposes. For example, a method involving bringing water, water vapor, or an inert gas into contact with a product under ambient pressure or reduced pressure may be used. In addition, there is also no particular limitation on an adsorption method that may be used in the step of providing a bio-derived ester compound in the present invention, and the adsorption method may be appropriately selected in accordance with purposes. For example, a method involving adding an adsorbent, such as activated white clay, activated carbon, magnesium silicate, aluminum silicate, aluminum phosphosilicate, magnesium hydroxide, aluminum hydroxide, magnesium oxide, aluminum oxide, zeolite, or silica gel, stirring the resultant or leaving the resultant to stand still as required, and then removing the adsorbent by a method such as filtration may be used.

In the present invention, from the viewpoint of providing a bio-derived ester compound with particularly reduced odor, as the step of providing a bio-derived ester compound by using the bio-derived branched primary alcohol having 6 to 12 carbon atoms and the carboxylic acid-based compound having a hydrocarbon group having 1 to 22 carbon atoms or the anhydride thereof, a method of providing a bio-derived ester compound by causing the bio-derived branched primary alcohol having 6 to 12 carbon atoms and the carboxylic acid-based compound having a hydrocarbon group having 1 to 22 carbon atoms or the anhydride thereof to react with each other at from 60°C to 300°C for from 1 hour to 8 hours in the presence of a catalyst as required, and then purifying the product through reduced-pressure distillation and adsorption treatment is particularly preferably used.

In the present invention, through incorporation of the step of providing a bio-derived branched primary alcohol having 6 to 12 carbon atoms, the step comprising dimerizing one kind or two or more kinds selected from the group consisting of: a bio-derived linear primary alcohol having a linear alkyl group having 3 to 6 carbon atoms; a bio-derived linear primary alcohol having a linear alkenyl group having 3 to 6 carbon atoms; a bio-derived linear aldehyde having a linear alkyl group having 3 to 6 carbon atoms; and a bio-derived linear aldehyde having a linear alkenyl group having 3 to 6 carbon atoms, and the step of providing a bio-derived ester compound by using the resultant bio-derived branched primary alcohol and a carboxylic acid-based compound having a hydrocarbon group having 1 to 22 carbon atoms or an anhydride thereof, as described above, a bio-derived ester compound with reduced odor can be obtained as described in Examples.

The bio-derived ester compound of the present invention is a bio-derived ester compound produced by a method of producing a bio-derived ester compound comprising: the step of providing a bio-derived branched primary alcohol having 6 to 12 carbon atoms, the step comprising dimerizing one kind or two or more kinds selected from the group consisting of: a bio-derived linear primary alcohol having a linear alkyl group having 3 to 6 carbon atoms; a bio-derived linear primary alcohol having a linear alkenyl group having 3 to 6 carbon atoms; a bio-derived linear aldehyde having a linear alkyl group having 3 to 6 carbon atoms; and a bio-derived linear aldehyde having a linear alkenyl group having 3 to 6 carbon atoms; and the step of providing a bio-derived ester compound by using the resultant bio-derived branched primary alcohol having 6 to 12 carbon atoms and a carboxylic acid-based compound having a hydrocarbon group having 1 to 22 carbon atoms or an anhydride thereof, as described above. Here, the bio-derived ester compound of the present invention contains slight amounts of inevitable impurities derived from the raw materials. When the inevitable impurities are specified, the ester compound of the present invention can be distinguished from an ester compound obtained by a production method using a petroleum raw material or the like and an ester compound obtained by a production method using a bio-derived compound different from the method of the present invention. However, it is impossible and impractical to uniformly specify the kinds and contents of the inevitable impurities because the kinds and contents of contained impurities vary depending on the kind of a plant resource or the like for providing a bio-based raw material and the reaction path of the compound. Thus, the bio-derived ester compound of the present invention is specified by the production method having raw materials limited.

There is no particular limitation on the form of use and embodiments of the bio-derived ester compound of the present invention, and the bio-derived ester compound of the present invention may be used by, for example, being blended in a cosmetic, a detergent, a paint, a pharmaceutical, a food, or the like. In this case, there is no particular limitation on the content of the bio-derived ester compound in a composition, such as a cosmetic, a detergent, a paint, a pharmaceutical, or a food, containing the bio-derived ester compound of the present invention, and the content may be set to, for example, from 0.001% by mass to 70.0% by mass with respect to the total amount of the composition, and is preferably from 0.01% by mass to 50.0% by mass, more preferably from 0.05% by mass to 30.0% by mass, still more preferably from 0.10% by mass to 20.0% by mass with respect to the total amount of the composition. The bio-derived ester compound of the present invention has reduced odor, and hence can exhibit desired characteristics while preventing any adverse effects on fragrance even when incorporated into the composition.

Various ingredients for improving and modifying various characteristics (e.g., solubility, dispersibility, stability, a feeling of use, applicability, permeability, moisture retention, safety, a design property, optical characteristics, fragrance, and a whitening property) during storage, during use, and after use, depending on intended uses may each be blended into the composition containing the bio-derived ester compound of the present invention. Examples of such ingredient include a higher alcohol, a powder ingredient, a higher fatty acid, a moisturizing agent, a water-soluble polymer, a sequestering agent, water, an oily ingredient, a lower alcohol, a polyhydric alcohol, a monosaccharide, an oligosaccharide, a polysaccharide, an amino acid and a derivative thereof, an organic amine, a pH adjuster, a vitamin, a UV protection ingredient, an antioxidant, a thickener, a surfactant, and other blendable ingredients (e.g., a preservative, a blood circulation promoter, an antiphlogistic agent, an activator, a whitening agent, an antiseborrheic agent, an anti-inflammatory agent, various extracts, and plant and seaweed extracts), and one kind or two or more kinds thereof may be optionally blended.

Examples of the higher alcohol include: linear higher alcohols, such as lauryl alcohol, cetyl alcohol, stearyl alcohol, behenyl alcohol, myristyl alcohol, oleyl alcohol, and cetostearyl alcohol; and branched higher alcohols, such as monostearyl glycerin ether (batyl alcohol), 2-decyltetradecinol, lanolin alcohol, cholesterol, phytosterol, hexyldodecanol, isostearyl alcohol, and octyldodecanol, and one kind or two or more kinds thereof may be used.

Examples of the powder ingredient include: inorganic powders (e.g., talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, a metal salt of tungstic acid, magnesium, silica, zeolite, barium sulfate, calcined calcium sulfate, calcium phosphate, fluorine apatite, hydroxyapatite, ceramic powder, zinc myristate, calcium palmitate, aluminum stearate, and boron nitride); organic powders (e.g., polyamide resin powder, polyethylene powder, polymethyl methacrylate powder, polystyrene powder, copolymer resin powder of styrene and acrylic acid, benzoguanamine resin powder, polytetrafluoroethylene powder, and cellulose powder); inorganic white pigments (e.g., titanium dioxide and zinc oxide); inorganic red pigments (e.g., iron oxide and iron titanate); inorganic brown pigments (e.g., γ-iron oxide); inorganic yellow pigments (e.g., yellow iron oxide and yellow ocher); inorganic black pigments (e.g., black iron oxide and lower titanium oxide); inorganic purple pigments (e.g., manganese violet and cobalt violet); inorganic green pigments (e.g., chromium oxide, chromium hydroxide, and cobalt titanate); inorganic blue pigments (e.g., ultramarine blue and Prussian blue); pearl pigments (e.g., titanium oxide-coated mica, titanium oxide-coated bismuth oxychloride, titanium oxide-coated talc, colored titanium oxide-coated mica, bismuth oxychloride, and argentine); metal powder pigments (e.g., aluminum powder and copper powder); organic pigments, such as zirconium, barium, and aluminum lakes (e.g., organic pigments, such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404, and Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1); and natural colorants (e.g., chlorophyl and β-carotene), and one kind or two or more kinds thereof may be used.

Examples of the higher fatty acid include lauric acid, myristic acid, palmitic acid, stearic acid, behenic acid, oleic acid, undecylenic acid, tall oil fatty acid, isostearic acid, linoleic acid, linolenic acid, eicosapentaenoic acid (EPA), and docosahexaenoic acid (DHA), and one kind or two or more kinds thereof may be used.

Examples of the moisturizing agent include polyethylene glycol, xylitol, sorbitol, maltitol, chondroitin sulfate, hyaluronic acid, mucoitin sulfate, charonin sulfate, atelocollagen, cholesteryl-12-hydroxystearate, sodium lactate, a bile acid salt, a dl-pyrrolidonecarboxylic acid salt, short-chain soluble collagen, a diglycerin (EO)PO adduct, a *Rosa roxburghii* extract, an *Achillea millefolium* extract, and a *Melilotus officinalis* extract, and one kind or two or more kinds thereof may be used.

As a natural water-soluble polymer, there are given, for example: plant-based polymers (e.g., gum arabic, gum tragacanth, galactan, guar gum, carob gum, gum karaya, carrageenan, pectin, agar, a quince seed (*Pyrus cydonia*), algae colloid (brown alga extract), starch (rice, corn, potato, and wheat), and glycyrrhizic acid); microorganism-based polymers (e.g., xanthan gum, dextran, succinoglucan, pullulan, and gellan gum); and animal-based polymers (e.g., collagen, casein, albumin, and gelatin), and one kind or two or more kinds thereof may be used.

Examples of the water-soluble polymer include: starch-based polymers (e.g., carboxymethyl starch and methylhydroxypropyl starch); cellulose-based polymers (e.g., methylcellulose, ethylcellulose, methylhydroxypropylcellulose, hydroxyethylcellulose, sodium cellulose sulfate, hydroxypropylcellulose, carboxymethylcellulose, sodium carboxymethylcellulose, crystalline cellulose, and cellulose powder); alginic acid-based polymers (e.g., sodium alginate and alginic acid propylene glycol ester); vinyl-based polymers (e.g., polyvinyl alcohol, polyvinyl methyl ether, polyvinylpyrrolidone, and a carboxyvinyl polymer); polyoxyethylene-based polymers (e.g., a polyoxyethylene-polyoxypropylene copolymer obtained by using polyethylene glycol 20,000, 40,000, or 60,000 as a raw material); acrylic polymers (e.g., sodium polyacrylate, polyethyl acrylate, and polyacrylamide); polyethylene imine; and cationic polymers, and one kind or two or more kinds thereof may be used.

Examples of the sequestering agent include 1-hydroxyethane-1,1-diphosphonic acid, 1-hydroxyethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, and trisodium ethylenediamine hydroxyethyl triacetate, and one kind or two or more kinds thereof may be used.

Examples of the oily ingredient include an ester oil (excluding the bio-derived ester of the present invention), a hydrocarbon oil, and a silicone oil, and one kind or two or more kinds thereof may be used. Examples of the ester oil out of those oily ingredients include isopropyl myristate, cetyl octanoate, isononyl isononanoate, octyldodecyl myristate, isopropyl palmitate, butyl stearate, hexyl laurate, myristyl myristate, decyl oleate, cetyl lactate, myristyl lactate, lanolin acetate, isocetyl stearate, isocetyl isostearate, cholesteryl 12-hydroxystearate, glyceryl di-2-ethylhexanoate, a dipentaerythritol fatty acid ester, glyceryl monoisostearate, neopentyl glycol dicaprate, diisostearyl malate, glyceryl di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, glyceryl trimyristate, glyceryl tri-2-heptylundecanoate, castor oil fatty acid methyl ester, oleyl oleate, an acetoglyceride, diisobutyl adipate, N-lauroyl-L-glutamic acid-2-octyldodecyl ester, ethyl laurate, diisopropyl sebacate, triethylhexanoin, and glyceryl tri (caprylate/caprate), and one kind or two or more kinds thereof may be used. In addition, oils and fats each containing an ester oil, such as avocado oil, camellia oil, turtle oil, macadamia nut oil, corn oil, mink oil, olive oil, rapeseed oil, egg yolk oil, sesame oil, apricot kernel oil, wheat germ oil, sasanqua oil, castor oil, linseed oil, safflower oil, cottonseed oil, perilla oil, soybean oil, peanut oil, tea seed oil, kaya oil, rice bran oil, shinagiri oil, Japanese tung oil, jojoba oil, germ oil, a triglyceride, cacao butter, coconut oil, hydrogenated coconut oil, palm oil, palm kernel oil, Japan wax kernel oil, a hydrogenated oil, Japan wax, and hydrogenated castor oil, may be used.

Examples of the hydrocarbon oil include liquid paraffin, ozokerite, squalane, pristane, paraffin, isododecane, ceresin, squalene, vaseline, and microcrystalline wax, and one kind or two or more kinds thereof may be used.

Examples of the silicone oil include chain polysiloxanes (e.g., dimethylpolysiloxane, methylphenylpolysiloxane, and diphenylpolysiloxane), cyclic polysiloxanes (e.g., octamethylcyclotetrasiloxane, decamethylcyclopentasiloxane, and dodecamethylcyclohexasiloxane), a silicone resin that forms a three-dimensional network structure, a silicone rubber, and various modified polysiloxanes (amino-modified polysiloxane, polyether-modified polysiloxane, alkyl-modified polysiloxane, and fluorine-modified polysiloxane), and one kind or two or more kinds thereof may be used.

Examples of the lower alcohol include ethanol, propanol, isopropanol, isobutyl alcohol, and t-butyl alcohol, and one kind or two or more kinds thereof may be used.

Examples of the polyhydric alcohol include: dihydric alcohols (e.g., ethylene glycol, propylene glycol, 1,3-butylene glycol, and 1,2-hexanediol); trihydric alcohols (e.g., glycerin and trimethylolpropane); tetrahydric alcohols (e.g., pentaerythritol of 1,2,6-hexanetriol); pentahydric alcohols (e.g., xylitol); hexahydric alcohols (e.g., sorbitol and mannitol); polyhydric alcohol polymers (e.g., diethylene glycol, dipropylene glycol, triethylene glycol, polypropylene glycol, tetraethylene glycol, diglycerin, polyethylene glycol, triglycerin, tetraglycerin, and polyglycerin); alkyl ethers of dihydric alcohols (e.g., ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); ether esters of dihydric alcohols (e.g., ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); sugar alcohols (e.g., sorbitol, maltitol, maltotriose, mannitol, sucrose, erythritol, glucose, fructose, amylolysis sugars, maltose, xylitose, and alcohols prepared by reduction of amylolysis sugars); Glysolid; tetrahydrofurfuryl alcohol; POE-tetrahydrofurfuryl alcohol; POP-butyl ether; POP/POE-butyl ether; tripolyoxypropylene glycerin ether; POP-glycerin ether; POP-glycerin ether phosphoric acid; POP/POE-pentaneerythritol ether; and polyglycerin, and one kind or two or more kinds thereof may be used.

Examples of the monosaccharide include: trioses (e.g., D-glyceraldehyde and dihydroxyacetone); tetroses (e.g., D-erythrose, D-erythrulose, D-threose, and erythritol); pentoses (e.g., L-arabinose, D-xylose, L-lixose, D-arabinose, D-ribose, D-ribulose, D-xylulose, and L-xylulose); hexoses (e.g., D-glucose, D-talose, D-psicose, D-galactose, D-fructose, L-galactose, L-mannose, and D-tagatose); heptoses (e.g., aldoheptose and heptulose); octoses (e.g., octulose); deoxy sugars (e.g., 2-deoxy-D-ribose, 6-deoxy-L-galactose, and 6-deoxy-L-mannose); amino sugars (e.g., D-glucosamine, D-galactosamine, sialic acid, aminouronic acid, and muramic acid); and uronic acids (e.g., D-glucuronic acid, D-mannuronic acid, L-guluronic acid, D-galacturonic acid, and L-iduronic acid), and one kind or two or more kinds thereof may be used.

Examples of the oligosaccharide include sucrose, umbelliferose, lactose, planteose, isolychnoses, α,α-trehalose, raffinose, lychnoses, umbilicin, stachyose, and verbascose, and one kind or two or more kinds thereof may be used.

Examples of the polysaccharide include cellulose, a quince seed, chondroitin sulfate, starch, galactan, dermatan sulfate, glycogen, gum arabic, heparan sulfate, hyaluronic acid, gum tragacanth, keratan sulfate, chondroitin, xanthan gum, mucoitin sulfate, guar gum, dextran, keratosulfate, locust bean gum, succinoglucan, and charonin sulfate, and one kind or two or more kinds thereof may be used.

Examples of the amino acid include: neutral amino acids (e.g., threonine and cysteine); and basic amino acids (e.g., hydroxylysine). In addition, examples of the amino acid derivative include sodium acylsarcosine (sodium lauroylsarcosine), acylglutamic acid salts, sodium acyl β-alanine, glutathione, and pyrrolidone carboxylic acid, and one kind or two or more kinds thereof may be used.

Examples of the organic amine include monoethanolamine, diethanolamine, triethanolamine, morpholine, triisopropanolamine, 2-amino-2-methyl-1,3-propanediol, and 2-amino-2-methyl-1-propanol, and one kind or two or more kinds thereof may be used.

Examples of the pH adjuster include buffers, such as lactic acid-sodium lactate, citric acid-sodium citrate, and succinic acid-sodium succinate, and one kind or two or more kinds thereof may be used.

Examples of the vitamin include vitamins A, B1, B2, B6, C, and E, and derivatives thereof, pantothenic acid and a derivative thereof, and biotin, and one kind or two or more kinds thereof may be used.

An inorganic UV protection ingredient, such as a powder pigment or a metal powder pigment, and a surface-treated product thereof, and an organic UV protection ingredient may each be used as the UV protection ingredient. Examples thereof include: metal oxides, such as titanium oxide, zinc oxide, cerium oxide, low-valence titanium oxide, and iron-doped titanium oxide; metal hydroxides such as iron hydroxide; metal flakes, such as plate-like iron oxide and aluminum flakes; ceramics such as silicon carbide; and fluorine compound-treated, silicone-treated, silicone resin-treated, pendant-treated, silane coupling agent-treated, titanium coupling agent-treated, silane-treated, oil-treated, N-acylated lysine-treated, polyacrylic acid-treated, metal soap-treated, acrylic resin-treated, and metal oxide-treated products thereof; and p-aminobenzoic acid-based UV protection ingredients, benzophenone-based UV protection ingredients, cinnamic acid-based UV protection ingredients, and benzoylmethane-based UV protection ingredients, 2-cyano-3,3-diphenylprop-2-enoic acid 2-ethylhexyl ester, 2-ethylhexyl dimethoxybenzylidene dioxoimidazolidinepropionate, 1-(3,4-dimethoxyphenyl)-4,4-dimethyl-1,3-pentanedione, cinoxate, methyl-O-aminobenzoate, 2-ethylhexyl-2-cyano-3,3-diphenylacrylate, 3-(4-methylbenzylidene)camphor, octyltriazone, 2-ethylhexyl 4-(3,4-dimethoxyphenylmethylene)-2,5-dioxo-1-imidazolidinepropionate, and polymer derivatives thereof, and one kind or two or more kinds thereof may be used.

Examples of the antioxidant include tocopherols, dibutylhydroxytoluene, butylhydroxyanisole, and gallic acid esters, and one kind or two or more kinds thereof may be used.

Examples of the thickener include xanthan gum, carrageenan, high-methoxyl pectin, low-methoxyl pectin, guar gum, gum arabic, crystalline cellulose, arabinogalactan, karaya gum, gum tragacanth, alginic acid, albumin, casein, curdlan, β-glucan, a β-glucan derivative, gellan gum, dextran, α-glucose and an α-glucose derivative, cellulose or a derivative thereof, keratin and collagen or derivatives thereof, calcium alginate, pullulan, agar, gelatin, *Tamarindus Indica* seed polysaccharide, a carbomer, a dimethyldiallylammonium chloride/acrylamide copolymer, dimethyldiallylammounium chloride hectorite, an acrylamide/acrylic acid/dimethyldiallylammonium chloride copolymer, and dibutylethylhexanoyl glutamide, and one kind or two or more kinds thereof may be used.

Examples of the surfactant include: cationic surfactants (e.g., lauryltrimethylammonium chloride, cetyltrimethylammonium chloride, stearyltrimethylammonium chloride, an alkyltrimethylammonium chloride, distearyldimethylammonium chloride, stearyltrimethylammonium saccharin, cetyltrimethylammonium saccharin, behenyltrimethylammonium methylsulfate, behenyldimethylamine, behenic acid diethylaminoethylamide, behenic acid dimethylaminopropylamide, behenic acid dimethylaminoethylamide, stearyldimethylamine, palmitoxypropyldimethylamine, and stearoxypropyldimethylamine); anionic surfactants (e.g., an alkyl ether sulfuric acid salt, an alkyl sulfuric acid salt, an alkyl ether sulfuric acid ester salt, an alkenyl ether sulfuric acid salt, an alkenyl sulfuric acid salt, an olefin sulfonic acid salt, an alkane sulfonic acid salt, a saturated or unsaturated fatty acid salt, an alkyl or alkenyl ether carboxylic acid salt, an α-sulfone fatty acid salt, an N-acyl amino acid-type surfactant, a phosphoric acid mono- or diester-type surfactant, a sulfosuccinic acid ester, an N-alkyloyl methyl taurine salt, and derivatives thereof); amphoteric surfactants (e.g.: a betaine-type amphoteric surfactant, such as cocamidopropyldimethyl betaine acetate, lauryldimethylamino acid betaine, a 2-alkyl-N-carboxymethyl-N-hydroxymethyl imidazolinium betaine, lauryl hydroxy sulfobetaine, lauroyl amide ethyl hydroxyethyl carboxymethyl betaine, or a metal salt of hydroxypropyl phosphoric acid; an amino acid-type amphoteric surfactant such as a metal salt of β-laurylamino propionic acid; a sulfuric acid ester-type amphoteric surfactant; and a sulfonic acid-type amphoteric surfactant); and non-ionic surfactants (e.g., POE cetyl ether (ceteth), POE stearyl ether (steareth), POE behenyl ether, POE oleyl ether (oleth), POE lauryl ether (laureth), POE octyldodecyl ether, POE hexyldecyl ether, POE isostearyl ether, POE nonylphenyl ether, POE octylphenyl ether, POE polyoxypropylene cetyl ether, POE polyoxypropylene decyltetradecyl ether, POE sorbitan monooleate, POE sorbitan monostearate, POE sorbitan monopalmitate, POE sorbitan monolaurate, POE sorbitan trioleate, POE glycerin monostearate, POE glycerin monomyristate, POE sorbit tetraoleate, POE sorbit hexastearate, POE sorbit monolaurate, POE sorbit beeswax, polyethylene glycol monooleate, polyethylene glycol monostearate, polyethylene glycol monolaurate, lipophilic glycerin monooleate, lipophilic glycerin monostearate, self-emulsifiable glycerin monostearate, sorbitan monooleate, sorbitan sesquioleate, sorbitan trioleate, sorbitan monostearate, sorbitan monopalmitate, sorbitan monolaurate, a sucrose fatty acid ester, decaglyceryl monolaurate, decaglyceryl monostearate, decaglyceryl monooleate, decaglyceryl monomyristate, an alkyl glucoside, POE methyl glucoside, and POE methyl glucoside dioleate), and one kind or two or more kinds thereof may be used.

Examples of the other blendable ingredients include: preservatives (e.g., methylparaben, ethylparaben, butylparaben, and phenoxyethanol); antiphlogistic agents (e.g., a glycyrrhizic acid derivative, a glycyrrhetinic acid derivative, hinokitiol, zinc oxide, and allantoin); whitening agents (e.g., a *Saxifraga sarmentosa* extract and arbutin); various extracts (e.g., phellodendron bark, *Coptis japonica, Lithospermum erythrorhizon* root, *Paeonia lactiflora, Swertia japonica,* birch, sage, *Eriobotrya japonica,* ginseng, aloe, mallow, iris, grape, coix seed, *Luffa cylindrica,* lily, saffron, *Cnidium officinale,* ginger, *Hypericum erectum, Ononis spinosa,* garlic, capsicum, *Citrus unshiu* peel, *Angelica acutiloba,* and seaweed extracts); activators (e.g., royal jelly, a photosensitive element, and a cholesterol derivative); blood circulation promoters (e.g., nicotinic acid benzyl ester, nicotinic acid β-butoxyethyl ester, capsaicin, zingerone, cantharides tincture, ichthammol, tannic acid, α-borneol, tocopherol nicotinate, inositol hexanicotinate, cyclandelate, cinnarizine, tolazoline, acetylcholine, verapamil, cepharanthine, and γ-orizanol); antiseborrheic agents (e.g., sulfur and thianthol); and anti-inflammatory agents (e.g., tranexamic acid, thiotaurine, and hypotaurine), and one kind or two or more kinds thereof may be used.

There is no particular limitation on the content at the time of the addition of each of the higher alcohol, the powder ingredient, the higher fatty acid, the moisturizing agent, the water-soluble polymer, the sequestering agent, the lower alcohol, the water, the polyhydric alcohol, the monosaccharide, the oligosaccharide, the polysaccharide, the amino acid and the derivative thereof, the organic amine, the pH adjuster, the vitamin, the UV protection ingredient, the antioxidant, the thickener, the surfactant, and the other blendable ingredients (e.g., the preservative, the blood circulation promoter, the antiphlogistic agent, the activator, the whitening agent, the antiseborrheic agent, the anti-inflammatory agent, the various extracts, and the plant and seaweed extracts) in the composition containing the bio-derived ester compound of the present invention, and the content may be adjusted in accordance with embodiments and purposes. For example, each of the above-mentioned additives may be added in an amount of from 0.001% by mass to 50.0% by mass with respect to the total mass of the composition.

In addition, the bio-derived ester compound of the present invention may also be used as an intermediate compound for providing a target compound through a reaction with any other raw material compound. There is no particular limitation on the other raw material compound that may be used in this case as long as the compound reacts with the bio-derived ester compound of the present invention. Examples thereof include compounds each having a functional group, such as an aldehyde group or a ketone group, in a molecule thereof. In addition, there is also no particular limitation on the target compound to be obtained by such method, and the target compound may be appropriately selected in accordance with purposes. In this case, for example, when p-methoxybenzaldehyde is used as the other raw material compound, a methoxycinnamic acid ester may be obtained as the target compound. More specifically, 2-ethylhexyl methoxycinnamate may be obtained by, for example, using bio-derived 2-ethylhexyl acetate that is a bio-derived ester compound as an intermediate compound and p-methoxybenzaldehyde as the other raw material compound and causing the bio-derived 2-ethylhexyl acetate and the p-methoxybenzaldehyde to react with each other by a known method. The target compound to be obtained by such method uses a bio-derived ester compound with reduced odor as an intermediate compound, and hence the target compound to be obtained may also be used as a compound with reduced odor. In addition, there is no particular limitation on the form of use and embodiments of the target compound obtained in this manner, and the target compound may be used by, for example, being blended in a cosmetic, a detergent, a paint, a pharmaceutical, a food, or the like in the same manner as in the above-mentioned bio-derived ester compound.

In this description, the following inventions are disclosed.
(1) A method of producing a bio-derived ester compound, comprising: a step of providing a bio-derived branched primary alcohol having a branched alkyl group having 6 to 12 carbon atoms, the step comprising dimerizing one kind or two or more kinds selected from the group consisting of: a bio-derived linear primary alcohol having a linear alkyl group having 3 to 6 carbon atoms; a bio-derived linear primary alcohol having a linear alkenyl group having 3 to 6 carbon atoms; a bio-derived linear aldehyde having a linear alkyl group having 3 to 6 carbon atoms; and a bio-derived linear aldehyde having a linear alkenyl group having 3 to 6 carbon atoms; and a step of providing a bio-derived ester compound by using the resultant bio-derived branched primary alcohol and a carboxylic acid-based compound having a hydrocarbon group having 1 to 22 carbon atoms or an anhydride thereof.
(2) The method of producing a bio-derived ester compound according to Item (1), comprising a step of providing a bio-derived branched primary alcohol having a branched alkyl group having 6 to 12 carbon atoms, the step comprising dimerizing one kind or two or more kinds selected from the group consisting of a bio-derived linear primary alcohol having a linear alkyl group having 3 to 6 carbon atoms.
(3) The method of producing a bio-derived ester compound according to Item (1) or (2), wherein the carboxylic acid-based compound having a hydrocarbon group having 1 to 22 carbon atoms or the anhydride thereof is one kind or two or more kinds selected from the group consisting of: an aliphatic monocarboxylic acid compound consisting of a linear saturated aliphatic hydrocarbon group having 10 to 18 carbon atoms and one carboxy group; and an aromatic monocarboxylic acid compound consisting of an aromatic hydrocarbon group having 6 to 12 carbon atoms, one carboxy group, and one hydroxy group.
(4) The method of producing a bio-derived ester compound according to any one of Items (1) to (3), wherein the carboxylic acid-based compound having a hydrocarbon group having 1 to 22 carbon atoms or the anhydride thereof to be used is one kind or two or more kinds selected from the group consisting of: lauric acid; myristic acid; palmitic acid; stearic acid; and salicylic acid.
(5) A bio-derived ester compound, which is produced by the production method of any one of Items (1) to (4).
(6) A cosmetic or a detergent, comprising the bio-derived ester compound of Item (5).
(7) A method of reducing an odor of an ester compound, comprising causing a bio-derived branched primary alcohol having a branched alkyl group having 6 to 12 carbon atoms to react as a raw material, wherein the bio-derived branched primary alcohol is obtained through a step of dimerizing one kind or two or more kinds selected from the group consisting of: a bio-derived linear primary alcohol having a linear alkyl group having 3 to 6 carbon atoms; a bio-derived linear primary alcohol having a linear alkenyl group having 3 to 6 carbon atoms; a bio-derived linear aldehyde having a linear alkyl group having 3 to 6 carbon atoms; and a bio-derived linear aldehyde having a linear alkenyl group having 3 to 6 carbon atoms, and wherein the ester compound is obtained by causing the bio-derived branched primary alcohol and a carboxylic acid-based compound having a hydrocarbon group having 1 to 22 carbon atoms or an anhydride thereof to react with each other.

### Examples

The present invention is specifically described below by way of the Examples. However, the present invention is by no means limited by the Examples, and modifications may be made without departing from the scope of the present invention. In the following Examples and the like, the term "%" is by mass unless otherwise stated.

### <Example 1>

3,552 g of bio-derived n-butanol, 424 g of tripotassium phosphate, 448 g of calcium oxide, and 160 g of copper powder were loaded into an autoclave, and then the mixture was subjected to a reaction at 290°C for 5 hours. The resultant was further filtered and distilled to provide 1,248 g of bio-derived 2-ethylhexanol that was a bio-derived branched primary alcohol in which the bio-derived n-butanol was dimerized, and 1,812 g of unreacted bio-derived n-butanol.

Subsequently, 104 g of the resultant bio-derived 2-ethylhexanol and 103 g of palmitic acid derived from a petroleum raw material were loaded into another reaction vessel and caused to react with each other at 190°C for 20 hours to provide 2-ethylhexyl palmitate in the reaction vessel. The resultant filtrate was decompressed to 4.0 kPa or less at from 110°C to 125°C so that unreacted bio-derived 2-ethylhexanol was removed. Then, 4.4 g of activated white clay was added to the residue, followed by stirring at 80°C for 30 minutes. After the resultant was cooled to room temperature, the activated white clay was filtered out. Thus, 137 g of bio-derived 2-ethylhexyl palmitate A was obtained.

### <Example 2>

80 g of the bio-derived 2-ethylhexanol obtained in Example 1, 78 g of palmitic acid derived from a petroleum raw material, and 1.6 g of 98% sulfuric acid serving as a catalyst were loaded into another reaction vessel and caused to react with each other at 90°C for 5 hours to provide 2-ethylhexyl palmitate in the reaction vessel. After the resultant was cooled to room temperature, the resultant liquid mixture was transferred to an oil-water separation machine, and 110 g of water and 2.6 g of a 48% sodium hydroxide aqueous solution were added thereto, followed by stirring at 60°C for 30 minutes. After that, the mixture was allowed to stand still for 30 minutes to be separated into an aqueous phase serving as a lower layer and an oil phase serving as an upper layer. Then, the oil phase was extracted and dehydrated under reduced pressure at 60°C and 27 kPa for 1 hour. Subsequently, 0.9 g of magnesium silicate and 0.9 g of aluminum silicate were added thereto, followed by stirring at 80°C for 30 minutes. After the resultant was cooled to room temperature, the magnesium silicate and the aluminum silicate were filtered out. The resultant filtrate was treated under reduced pressure at from 110°C to 125°C and 4.0 kPa or less to remove unreacted bio-derived 2-ethylhexanol. After that, 6.6 g of activated white clay was added to the residue, followed by stirring at 80°C for 30 minutes. After the resultant was cooled to room temperature, the activated white clay was filtered out. Thus, 101 g of bio-derived 2-ethylhexyl palmitate B was obtained.

### <Example 3>

130 g of the bio-derived 2-ethylhexanol obtained in Example 1, 55 g of salicylic acid derived from a petroleum raw material, and 3.9 g of 98% sulfuric acid serving as a catalyst were loaded into a reaction vessel and caused to react with each other at 90°C for 24 hours to provide 2-ethylhexyl salicylate in the reaction vessel. After the resultant was cooled to room temperature, the resultant liquid mixture was transferred to an oil-water separation machine, and 50 g of water and 2.4 g of a 48% sodium hydroxide aqueous solution were added thereto, followed by stirring at 60°C for 30 minutes. After that, the mixture was allowed to stand still for 30 minutes to be separated into an aqueous phase serving as a lower layer and an oil phase serving as an upper layer. Then, the oil phase was extracted and dehydrated under reduced pressure at 60°C and 27 kPa for 1 hour. Subsequently, 0.8 g of magnesium silicate and 0.8 g of aluminum silicate were added thereto, followed by stirring at 80°C for 30 minutes. After the resultant was cooled to room temperature, the magnesium silicate and the aluminum silicate were filtered out. The resultant filtrate was treated under reduced pressure at from 110°C to 125°C and 4.0 kPa or less to remove unreacted bio-derived 2-ethylhexanol. After that, 3 g of activated white clay was added to the residue, followed by stirring at 80°C for 30 minutes. After the resultant was cooled to room temperature, the activated white clay was filtered out. Thus, 85 g of bio-derived 2-ethylhexyl salicylate A was obtained.

### <Example 4>

130 g of the bio-derived 2-ethylhexanol obtained in Example 1, 55 g of salicylic acid derived from a petroleum raw material, and 0.28 g of zirconium octoate serving as a catalyst were loaded into a reaction vessel and caused to react with each other at 200°C for 8 hours to provide 2-ethylhexyl salicylate in the reaction vessel. After the resultant was cooled to room temperature, 3.4 g of aluminum silicate was added thereto, followed by stirring at 80°C for 30 minutes. After the resultant was cooled to room temperature, the aluminum silicate was filtered out. The resultant filtrate was treated under reduce pressure at from 110°C to 125°C and 4.0 kPa or less to remove unreacted bio-derived 2-ethylhexanol. Then, 3 g of activated white clay was added to the residue, followed by stirring at 80°C for 30 minutes. After the resultant was cooled to room temperature, the activated white clay was filtered out. Thus, 90 g of bio-derived 2-ethylhexyl salicylate B was obtained.

### <Example 5>

104 g of the bio-derived 2-ethylhexanol obtained in Example 1 and 103 g of bio-derived palmitic acid were loaded into another reaction vessel and caused to react with each other at 190°C for 20 hours to provide 2-ethylhexyl palmitate in the reaction vessel. The resultant filtrate was subject to reduced pressure at 4.0 kPa or less at from 110°C to 125°C so that unreacted bio-derived 2-ethylhexanol was removed. Then, 4.4 g of activated white clay was added to the residue, followed by stirring at 80°C for 30 minutes. After the resultant was cooled to room temperature, the activated white clay was filtered out. Thus, 137 g of bio-derived 2-ethylhexyl palmitate C was obtained.

### <Comparative Example 1>

Petroleum raw material-derived 2-ethylhexyl palmitate A' was obtained by the same method as in Example 1 except that 104 g of petroleum raw material-derived 2-ethylhexanol, which had been produced by producing n-butyraldehyde from propylene derived from a petroleum raw material by an oxo method, and then performing the aldol condensation and hydrogenation of the n-butyraldehyde, was used instead of the bio-derived 2-ethylhexanol.

### <Comparative Example 2>

Petroleum raw material-derived 2-ethylhexyl palmitate B' was obtained by the same method as in Example 2 except that 80 g of petroleum raw material-derived 2-ethylhexanol, which had been produced by producing n-butyraldehyde from propylene derived from a petroleum raw material by an oxo method, and then performing the aldol condensation and hydrogenation of the n-butyraldehyde, was used instead of the bio-derived 2-ethylhexanol.

### <Comparative Example 3>

Petroleum raw material-derived 2-ethylhexyl salicylate A' was obtained by the same method as in Example 3 except that 130 g of petroleum raw material-derived 2-ethylhexanol, which had been produced by producing n-butyraldehyde from propylene derived from a petroleum raw material by an oxo method, and then performing the aldol condensation and hydrogenation of the n-butyraldehyde, was used instead of the bio-derived 2-ethylhexanol.

### <Comparative Example 4>

Petroleum raw material-derived 2-ethylhexyl salicylate B' was obtained by the same method as in Example 4 except that 130 g of petroleum raw material-derived 2-ethylhexanol, which had been produced by producing n-butyraldehyde from propylene derived from a petroleum raw material by an oxo method, and then performing the aldol condensation and hydrogenation of the n-butyraldehyde, was used instead of the bio-derived 2-ethylhexanol.

### <Comparative Example 5>

Petroleum raw material-derived 2-ethylhexyl palmitate C' was obtained by the same method as in Example 5 except that 104 g of petroleum raw material-derived 2-ethylhexanol, which had been produced by producing n-butyraldehyde from propylene derived from a petroleum raw material by an oxo method, and then performing the aldol condensation and hydrogenation of the n-butyraldehyde, was used instead of the bio-derived 2-ethylhexanol.

### <Evaluation of Odor>

The ester compounds produced in Examples 1 to 5 and Comparative Examples 1 to 5 were each evaluated for its odor immediately after the production. Specifically, seven panelists each checked the odor of each of the produced ester compounds and scored 10 samples on a 4-point scale of from 1 to 4, with 4 points being given for the sample with the lowest odor. The total score of the score results of the respective panelists was calculated, and the odor was evaluated as follows: when the total score was 24 points or more, the odor was evaluated as "OO" (excellent); when the total score was 18 points or more and less than 24 points, the odor was evaluated as "O" (good); and when the total score was less than 18 points, the odor was evaluated as "X" (bad). The evaluation results are shown in Table 1. The total score of less than 18 points in this evaluation indicates that the resultant ester compound is poor in odor and hence lacks practicality.

**Table 1**

| | | Evaluation of odor | |
|---|---|---|---|
| | | Total score | Evaluatio n result |
| Example 1 | Bio-derived 2-ethylhexyl palmitate A | 20 | O |
| Example 2 | Bio-derived 2-ethylhexyl palmitate B | 27 | OO |
| Example 3 | Bio-derived 2-ethylhexyl salicylate A | 28 | OO |
| Example 4 | Bio-derived 2-ethylhexyl salicylate B | 20 | O |
| Example 5 | Bio-derived 2-ethylhexyl palmitate C | 20 | O |
| Comparative Example 1 | Petroleum raw material-derived 2-ethylhexyl palmitate A' | 7 | X |
| Comparative Example 2 | Petroleum raw material-derived 2-ethylhexyl palmitate B' | 16 | X |
| Comparative Example 3 | Petroleum raw material-derived 2-ethylhexyl salicylate A' | 14 | X |
| Comparative Example 4 | Petroleum raw material-derived 2-ethylhexyl salicylate B' | 8 | X |
| Comparative Example 5 | Petroleum raw material-derived 2-ethylhexyl palmitate C' | 7 | X |

According to the present invention, a bio-derived ester compound with reduced odor can be obtained by using a bio-derived raw material.

## Claims

1. A method of producing a bio-derived ester compound, comprising:
a step of providing a bio-derived branched primary alcohol having a branched alkyl group having 6 to 12 carbon atoms, the step comprising dimerizing one kind or two or more kinds selected from the group consisting of: a bio-derived linear primary alcohol having a linear alkyl group having 3 to 6 carbon atoms; a bio-derived linear primary alcohol having a linear alkenyl group having 3 to 6 carbon atoms; a bio-derived linear aldehyde having a linear alkyl group having 3 to 6 carbon atoms; and a bio-derived linear aldehyde having a linear alkenyl group having 3 to 6 carbon atoms; and
a step of providing a bio-derived ester compound by using the resultant bio-derived branched primary alcohol and a carboxylic acid-based compound having a hydrocarbon group having 1 to 22 carbon atoms or an anhydride thereof.

2. The method of producing a bio-derived ester compound according to claim 1, comprising a step of providing a bio-derived branched primary alcohol having a branched alkyl group having 6 to 12 carbon atoms, the step comprising dimerizing one kind or two or more kinds selected from the group consisting of a bio-derived linear primary alcohol having a linear alkyl group having 3 to 6 carbon atoms.

3. The method of producing a bio-derived ester compound according to claim 1, wherein the carboxylic acid-based compound having a hydrocarbon group having 1 to 22 carbon atoms or the anhydride thereof is one kind or two or more kinds selected from the group consisting of: an aliphatic monocarboxylic acid compound consisting of a linear saturated aliphatic hydrocarbon group having 10 to 18 carbon atoms and one carboxy group; and an aromatic monocarboxylic acid compound consisting of an aromatic hydrocarbon group having 6 to 12 carbon atoms, one carboxy group, and one hydroxy group.

4. The method of producing a bio-derived ester compound according to claim 2, wherein the carboxylic acid-based compound having a hydrocarbon group having 1 to 22 carbon atoms or the anhydride thereof is one kind or two or more kinds selected from the group consisting of: an aliphatic monocarboxylic acid compound consisting of a linear saturated aliphatic hydrocarbon group having 10 to 18 carbon atoms and one carboxy group; and an aromatic monocarboxylic acid compound consisting of an aromatic hydrocarbon group having 6 to 12 carbon atoms, one carboxy group, and one hydroxy group.

5. The method of producing a bio-derived ester compound according to claim 1, wherein the carboxylic acid-based compound having a hydrocarbon group having 1 to 22 carbon atoms or the anhydride thereof to be used is one kind or two or more kinds selected from the group consisting of: lauric acid; myristic acid; palmitic acid; stearic acid; and salicylic acid.

6. The method of producing a bio-derived ester compound according to claim 2, wherein the carboxylic acid-based compound having a hydrocarbon group having 1 to 22 carbon atoms or the anhydride thereof to be used is one kind or two or more kinds selected from the group consisting of: lauric acid; myristic acid; palmitic acid; stearic acid; and salicylic acid.

7. A bio-derived ester compound, which is produced by the production method of any one of claims 1 to 6.

8. A cosmetic or a detergent, comprising the bio-derived ester compound of claim 7.

9. A method of reducing an odor of an ester compound, comprising causing a bio-derived branched primary alcohol having a branched alkyl group having 6 to 12 carbon atoms to react as a raw material,
wherein the bio-derived branched primary alcohol is obtained through a step of dimerizing one kind or two or more kinds selected from the group consisting of: a bio-derived linear primary alcohol having a linear alkyl group having 3 to 6 carbon atoms; a bio-derived linear primary alcohol having a linear alkenyl group having 3 to 6 carbon atoms; a bio-derived linear aldehyde having a linear alkyl group having 3 to 6 carbon atoms; and a bio-derived linear aldehyde having a linear alkenyl group having 3 to 6 carbon atoms, and
wherein the ester compound is obtained by causing the bio-derived branched primary alcohol and a carboxylic acid-based compound having a hydrocarbon group having 1 to 22 carbon atoms or an anhydride thereof to react with each other.
